(19) Europäisches Patentamt
European Patent Office
Office européen des brevets

(11) Publication number: **0 040 944 A1**

# (12) EUROPEAN PATENT APPLICATION

(21) Application number: **81302197.9**

(22) Date of filing: **18.05.81**

(51) Int. Cl.³: **C 07 D 307/93**, C 07 D 333/78, A 61 K 31/34, A 61 K 31/38

(30) Priority: **20.05.80 JP 67228/80**

(43) Date of publication of application: **02.12.81 Bulletin 81/48**

(84) Designated Contracting States: **BE CH DE FR GB IT LI NL SE**

(71) Applicant: **SUMITOMO CHEMICAL COMPANY, LIMITED, 15 Kitahama 5-chome Higashi-ku, Osaka-shi Osaka-fu (JP)**

(72) Inventor: **Inokuma, Shun, 2-10-1-146, Sonehigashi-machi, Toyonaka Osaka (JP)**
Inventor: **Sugie, Akihiko, 2-10-1-116, Sonehigashi-machi, Toyonaka Osaka (JP)**
Inventor: **Shimomura, Hiromi, c/o Shinei Heights Shinano 2C, 27-1, Shinano-machi Niigata (JP)**
Inventor: **Katsube, Junki, 10-20, Machikaneyama-cho, Toyonaka Osaka (JP)**

(74) Representative: **Allard, Susan Joyce et al, BOULT, WADE & TENNANT 27 Furnival street, London EC4A 1PQ (GB)**

(54) **Heterotricyclic cage compounds, processes for their production, pharmaceutical compositions containing them, and their use as medicinal antiviral agents.**

(57) Compounds of the formula:

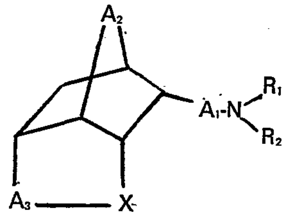

wherein $A_1$ is a single bond, a $C_2$-$C_5$ alkylene group or a $C_1$-$C_5$ alkylidene group, $A_2$ is a $C_2$-$C_3$ alkylene group or a $C_1$-$C_3$ alkylidene group, $A_3$ is a $C_2$-$C_4$ alkylene group or a $C_1$-$C_4$ alkylidene group, X is -O- or -$SO_n$- (wherein n is an integer of 0 to 2), $R_1$ is a hydrogen atom, a $C_1$-$C_5$ alkyl group, a $C_3$-$C_5$ alkenyl group and $R_2$ is a hydrogen atom, a $C_1$-$C_5$ alkyl group, a $C_3$-$C_5$ alkenyl group, a $C_2$-$C_5$ alkanoyl group, a $C_2$-$C_5$ alkoxycarbonyl group, a benzyloxycarbonyl group, or when taken together with the adjacent nitrogen atom, $R_1$ and $R_2$ may form a five or six-membered heterocyclic group, which are useful as antiviral agents.

EP 0 040 944 A1

ACTORUM AG

0040944

# HETEROTRICYCLIC CAGE COMPOUNDS, PROCESSES FOR THEIR PRODUCTION, PHARMACEUTICAL COMPOSITIONS CONTAINING THEM, AND THEIR USE AS MEDICINAL ANTIVIRAL AGENTS.

The present invention relates to tricyclic cage compounds, and their production and use. More particularly, this invention relates to novel heterotricyclic cage compounds, a pharmaceutical composition containing at least one of them and a process for preparing them.

The novel heterotricyclic cage compounds of this invention are those represented by the following formula (I):

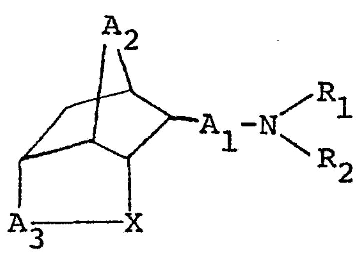

(I)

wherein $A_1$ is a single bond, a $C_2$-$C_5$ alkylene group or a $C_1$-$C_5$ alkylidene group, $A_2$ is a $C_2$-$C_3$ alkylene group or a $C_1$-$C_3$ alkylidene group, $A_3$ is a $C_2$-$C_4$ alkylene group or a $C_1$-$C_4$ alkylidene group, X is -O- or $-SO_n-$ (wherein n is an integer of 0 to 2), $R_1$ is a hydrogen atom, a $C_1$-$C_5$ alkyl group, a $C_3$-$C_5$ alkenyl group and $R_2$ is a hydrogen atom, a $C_1$-$C_5$ alkyl group, a $C_3$-$C_5$ alkenyl group, a $C_2$-$C_5$ alkanoyl group, a $C_2$-$C_5$ alkoxycarbonyl group or a benzyloxycarbonyl group, or when taken together with the adjacent nitrogen atom, $R_1$ and $R_2$ may form a five or six-membered heterocyclic group, and their non-toxic salts.

In the significances as used above, "$C_2$-$C_5$

alkylene" for $A_1$ means an alkylene group having 2 to 5 carbon atoms (e.g. ethylene, trimethylene, propylene); "$C_1$-$C_5$ alkylidene" for $A_1$ means an alkylidene group having 1 to 5 carbon atoms (e.g. methylene, ethylidene, propylidene, isopropylidene); "$C_2$-$C_3$ alkylene" for $A_2$ means an alkylene group having 2 or 3 carbon atoms (e.g. ethylene, trimethylene, cyclopropylene); "$C_1$-$C_3$ alkylidene" for $A_2$ means an alkylidene group having 1 to 3 carbon atoms (e.g. methylene, ethylidene, propylidene); "$C_2$-$C_4$ alkylene" for $A_3$ means an alkylene group having 2 to 4 carbon atoms (e.g. ethylene, trimethylene); "$C_1$-$C_4$ alkylidene" for $A_3$ means an alkylidene group having 1 to 4 carbon atoms (e.g. methylene, ethylidene). Further, "$C_1$-$C_5$ alkyl" for $R_1$ and $R_2$ includes methyl, ethyl, n-propyl, iso-butyl, etc.; "$C_3$-$C_5$ alkenyl" for $R_1$ and $R_2$ are allyl, crotyl, 3,3-dimethylallyl, etc.; "$C_2$-$C_5$ alkanoyl" for $R_2$ are acetyl, propionyl, butyryl, etc.; "$C_2$-$C_5$ alkoxycarbonyl" for $R_2$ means an alkoxycarbonyl group having 2 to 5 carbon atoms (e.g. methoxycarbonyl, ethoxycarbonyl, t-butoxycarbonyl); and a "five or six-membered heterocyclic group" for 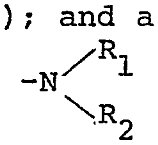 means a five or six-membered heterocyclic group having 3 to 5 carbon atoms (e.g. pyrrolidino, piperazino, morpholino).

Among the heterocyclic cage compounds (I) of the invention, those of the following formula (Ia) are preferable:

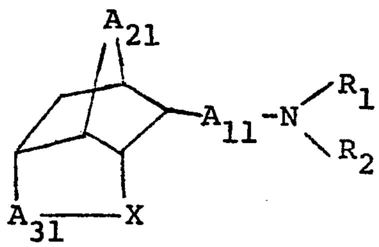

(Ia)

wherein $R_1$, $R_2$ and X are each as defined above, $A_{11}$ is a single bond or a methylene group, $A_{21}$ is a methylene group or an ethylene group and $A_{31}$ is a methylene group or an ethylene group.

Particularly preferred are those of the following formula (Ib):

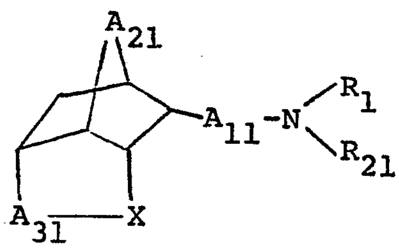

(Ib)

wherein $A_{11}$, $A_{21}$, $A_{31}$, X and $R_1$ are each as defined above and $R_{21}$ is a hydrogen atom, a $C_1$-$C_5$ alkyl group, a $C_3$-$C_5$ alkenyl group or a $C_2$-$C_5$ alkoxycarbonyl group.

More particularly preferred are those of the formula (Ic):

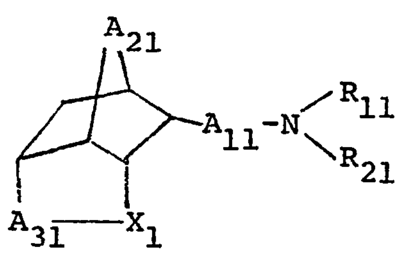

(Ic)

wherein $A_{11}$, $A_{21}$, $A_{31}$ and $R_{21}$ are each as defined above, $R_{11}$ is a hydrogen atom or a $C_1$-$C_5$ alkyl group and $X_1$ is -O- or -S-.

More preferred are those of the following formula

(Id):

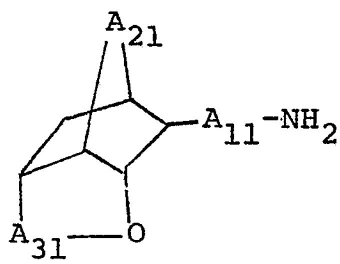

(Id)

wherein $A_{11}$, $A_{21}$ and $A_{31}$ are each as defined above.

Concerning heterotricyclic cage compounds having a skeleton of the formula:

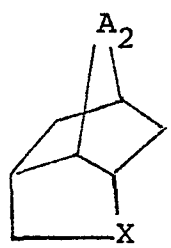

wherein $A_2$ and X are each as defined above, only a few compounds have hitherto been described, and few study on their chemistry or synthesis has hitherto been done. That is, the following compounds were synthesized previously:

(A) M. Nakazaki, K. Naemura and Y. Kondo: J. Org. Chem., 41, p. 1229 (1979) report the preparation of the compound of the formula:

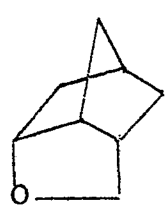

(B) R.M. Moriarty and K. Kapadia: Tetrahedron Letters, p. 1165 (1964) report the preparation of the compounds of the formulae:

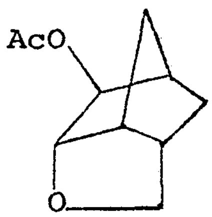

and

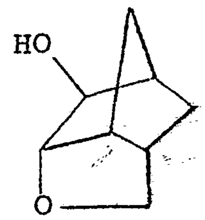

(C) R.M. Moriarty, H. Gopal and T. Adams: Tetrahedron Letters, p. 4003 (1970) report the preparation of the compounds of the formulae:

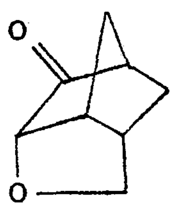

and

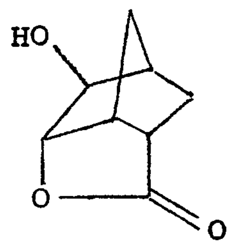

(D) R.M. Moriarty, C.R. Romain and T.O. Lovett: J. Am. Chem. Soc., 89, p. 3927 (1967) report the preparation of the compounds of the formulae:

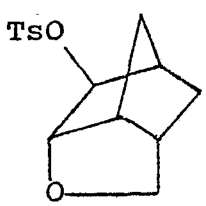

and

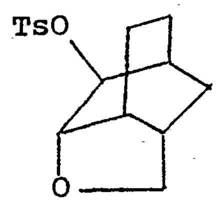

(E) C.R. Johnson and W.D. Kingsbury: J. Org. Chem., 38, p. 1803 (1973) report the preparation of the compounds of the formulae:

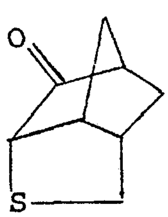

and

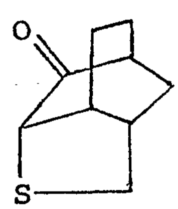

Under the situation as described above, the present inventors have studied and developed the synthesis of the said heterotricyclic cage compounds and also made search on their useful biological activities. As the

result, it has now been found that the novel heterotricyclic cage compounds (I) of the invention exhibit excellent antiviral activity.

As reviewed by L. Weinstein in "Pharmacological Basis of Therapeutics" (L.S. Goodman & A. Gilman, McMillian Company), p. 1305-1307 (1970), very few agents have been found to have clinical applicability for antiviral drug although the search for substances that might be of use in the management of viral infections has been long and intensive. Idoxuridine, amantadine and methiazone, synthetic antivirals for clinical use, are described in said text.

The heterotricyclic cage compound (I) of the invention may be employed to control, namely to treat and prevent viral infections caused by RNA type viruses such as Myxo group.

As a clinically useful anti-influenza A viral agent with a cage structure, amantadine of the following formula is already known (cf. Merck Index, 9th, p. 367-368):

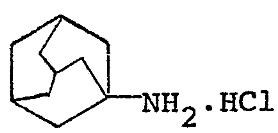

The amantadine is now marketed in U.S.A. as an anti-influenza A viral agent. However, it possesses not only an antiviral activity but also strong effects on a central nervous system (CNS). Based on such CNS-effects, amantadine is now marketed as an anti-parkinsonism agent in several countries including Japan, West Germany, United

Kingdom and France. For antiviral agents, the CNS-effects of amantadine is deemed as undesirable side effects.

The heterotricyclic cage compounds (I) of the present invention are found to be very effective to inhibit the growth of influenza virus, but surprisingly, exerting no or almost no CNS-effects. In addition, the toxicities of the heterotricyclic cage compound (I) are found to be significantly lower than those of amantadine. Thus, the heterotricyclic cage compounds (I) are very useful as antiviral agents with no or weak side effects or toxicities.

Some of the heterotricyclic cage compounds (I) of the invention are also useful as intermediates for another type of the heterotricyclic cage compounds (I).

The heterotricyclic cage compounds (I) and their non-toxic salts can be used for treatment of the viral infection of mammals caused by influenza virus or for prevention thereof. For this purpose, they can be administered either alone or in combination with pharmaceutically acceptable carriers. The proportion of the agent administered is determined by the solubility and chemical nature of the compound, chosen route of administration and standard biological practice. For example, they may be administered orally in solid forms, such as starch, mild sugar and so forth. They may be also administered orally in the form of solutions or injected parenterally. For parenteral administration, they may be used in the form of sterile solutions containing other solutes, for example,

enough saline or glucose to make the solutions isotonic.

The compounds (I) of the invention may be used in the form of pharmaceutical preparations appropriate for enteral or parenteral administration. Preferable excipients used therein are those which do not react with the compounds mentioned above, for example, water, gelatine, lactose, starch, stearic acid, magnesium stearate, talc, white petroleum jelly, vegetable oils, alcohol, benzyl alcohol, gums, polyalkylene glycols, or other known excipients for medicines. The pharmaceutical preparations may be in the form of tablets, powder, dragees (sugar coated tablets), capsules, suppositories, liquids, elixirs, emulsions, suspensions, syrups, chocolate, candy, waters, chewing gum or the like. If desired, they are sterilized and/or contain auxiliary substances such as preservatives, stabilizers, wetting agents, detergents or buffers. They may also additionally contain other therapeutically valuable substances (e.g. other antiviral agents, chemotherapeutic agents, antibiotics, anti-inflammatory agents, anti-pyretics, analgesics, enzyme preparations or the like).

The preparation of them are formulated by usual methods. They may be conjointly administered with a viral inhibitor such as interferon, interferon inducer or the like. The compositions and preparations should contain at least 0.1 % of active component.

The compositions of this invention may be administered enterally, parenterally or in the form of nasal

and oral aerosol spray. The compositions of this invention are also effective in resisting as well as combatting viral infections when administered topically to the site of the infection or potential infection in the form of ointments, salves, lotions, creams, sprays, drops, etc. The amount of the active component in such useful compositions or preparations is such that a suitable dosage of 0.5 mg to 50 mg/Kg/day will be obtained.

These compounds may be used systemically, more concretely, parenterally and non-parenterally, etc. and by a local application such as inhalation, topical application and dropping in the eye, etc., in the preparation form suitable for application such as liquid, ointment, powder, granule, mush, pellet, capsule, tablet, etc. by the addition of afore-mentioned solvent, additives, carriers, etc.

The heterotricyclic cage compounds (I) of this invention can be prepared by the following methods:

Method A

The compound of the formula (Ie):

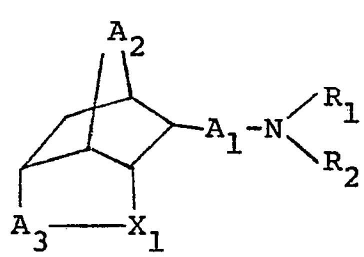

(Ie)

wherein $A_1$, $A_2$, $A_3$, $X_1$, $R_1$ and $R_2$ are each as defined above can be prepared from the corresponding tricyclic cage compound having a suitable substituent by either one of the following methods thereby converting the substituent into an

amino group represented by 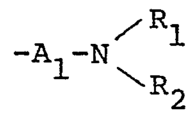 :

<u>(Aa) via Reduction</u>

(1) $-A_{12}-CN \longrightarrow -A_{12}-CH_2NH_2$

wherein $A_{12}$ is a single bond, a $C_2$-$C_4$ alkylene group or a $C_1$-$C_4$ alkylidene group;

(2) 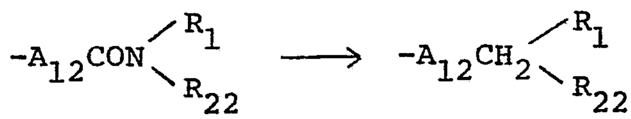

wherein $A_{12}$ and $R_1$ are each as defined above and $R_{22}$ is a hydrogen atom, a $C_1$-$C_5$ alkyl group or a $C_3$-$C_5$ alkenyl group, or when taken together with the adjacent nitrogen atom, $R_1$ and $R_{22}$ may form a five or six-membered heterocyclic group;

(3) 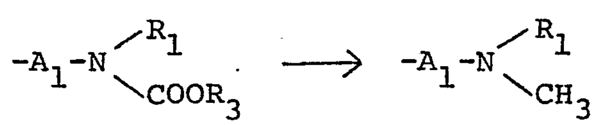

wherein $A_1$ and $R_1$ are each as defined above and $R_3$ is a $C_1$-$C_4$ alkyl group;

(4) 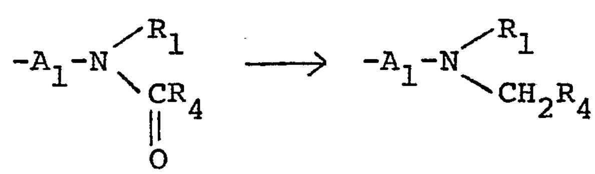

wherein $A_1$ and $R_1$ are each as defined above and $R_4$ is a hydrogen atom, a $C_1$-$C_4$ alkyl group or a $C_2$-$C_4$ alkenyl group;

(5) 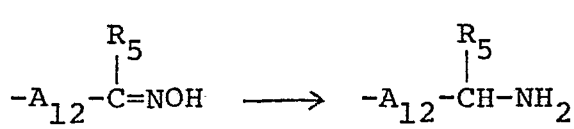

wherein $A_{12}$ is as defined above and $R_5$ is a hydrogen atom or a $C_1$-$C_4$ alkyl group;

(6) $-A_1-N_3 \longrightarrow -A_1-NH_2$

wherein $A_1$ is as defined above;

(7) 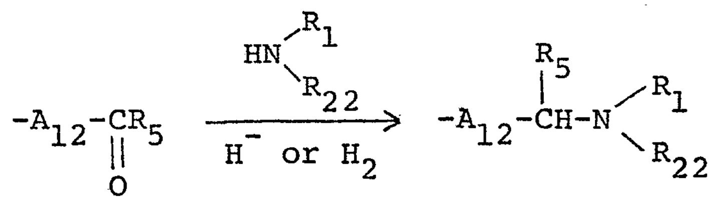


wherein $A_{12}$, $R_1$, $R_{22}$ and $R_5$ are each as defined above;

(8) 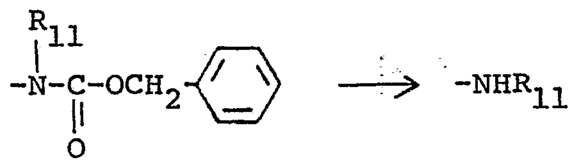

wherein $R_{11}$ is as defined above.

The reduction (1), (2), (3), (4), (5) or (6) may preferably be carried out by treatment with a metal hydride compound such as lithium aluminum hydride, sodium trialkoxyaluminum hydride, sodium diethylaluminum hydride or sodium bis(methoxyethoxy)aluminum hydride in an inert solvent such as ether, tetrahydrofuran or toluene.

The reaction (7) is known as the reductive amination, and it may be effected by reacting the carbonyl compound with ammonia or an amine in the presence of a reducing agent such as a metal borohydride, an active metal and an acid or formic acid or its derivative in a solvent such as methanol, ethanol, benzene or toluene.

The reduction (1), (5), (6) or (8) may be also effected by hydrogenation in the presence of a catalyst such as palladium charcoal, platinum oxide or Raney nickel in an inert solvent such as methanol, ethanol, benzene or toluene. The temperature for the above reduction may vary depending

on the type of reduction or reducing agent to be used therein, but these reactions (1) to (8) may generally be carried out at a temperature of from -20°C to the boiling point of the solvent to be used therein.

(Ab) via Substitution

(1) 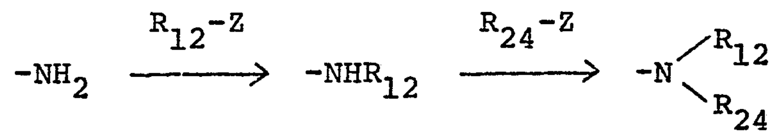


wherein Z is the residue of a strong acid, $R_{12}$ is the same as $R_1$ excluding a hydrogen atom and $R_{24}$ is the same as $R_2$ excluding a hydrogen atom;

(2) 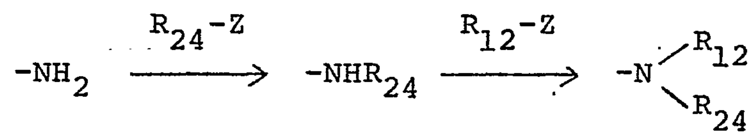


wherein Z, $R_{12}$ and $R_{24}$ are each as defined above;

(3) 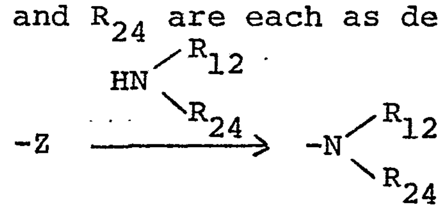

wherein Z, $R_{12}$ and $R_{24}$ are each as defined above.

The reaction (1) or (2) may be effected by reacting the amine compound ($-NH_2$, $-NHR_{12}$ or $-NHR_{24}$) with the activated radical such as $R_{12}$-Z or $R_{24}$-Z. The reaction (3) may also be achieved by reacting the compound of the formula (IIa):

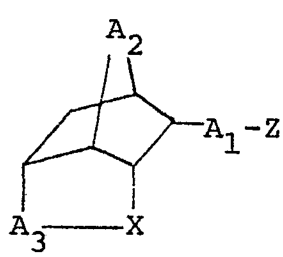

(IIa)

wherein $A_1$, $A_2$, $A_3$, X and Z are each as defined above, with the amine (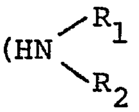).

Examples of the residue of a strong acid represented by Z are chloride (-Cl), bromide (-Br), p-toluenesulfonate ($-OSO_2-$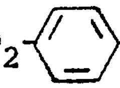$-CH_3$), methanesulfonate ($-OSO_2-CH_3$), sulfate ($-O-SO_2-O-$) and so on. The reaction (1), (2) or (3) may preferably be carried out by using an organic or inorganic base.

A variety of bases can be used in these reactions (1) to (3) (e.g. NaH, $NaOCH_3$, t-BuOK, NaOH, KOH, $Na_2CO_3$, $K_2CO_3$, $NaHCO_3$, triethylamine, pyridine, dimethylaniline). The base may preferably be used from 1 to 3 equivalent to the activated radical to be used therein.

In the reaction (1) or (2), the activated radical $R_{12}-Z$, $R_{24}-Z$ may preferably be used from 1 to 3 equivalent to the amine to be used therein, and in the reaction (3), the amine 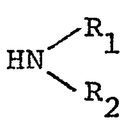 may preferably be used in an excess to the compound of the formula (IIa).

The reactions (1) to (3) can be carried out in the presence of an inert solvent (e.g. benzene, toluene, tetrahydrofuran, ether, dimethoxyethane, methanol, ethanol). The reaction conditions may vary depending upon the reaction temperature and the activated radicals to be used therein. The reaction temperature may be from 0°C to boiling point of the solvent used.

When each of $R_{12}$ and $R_{24}$ means an alkyl group, an alkenyl group, an alkynyl group or an aralkyl group, over-substitution may often occur giving a mixed product, and therefore in these cases, milder conditions may preferably be employed.

(Ac) via Hydrolysis

(1) 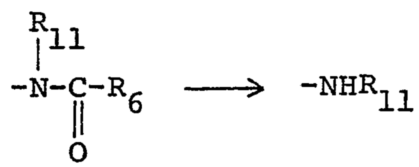

wherein $R_{11}$ is as defined above and $R_6$ is a hydrogen atom, a $C_1$-$C_4$ alkyl group, a $C_1$-$C_3$ haloalkyl group, a $C_1$-$C_4$ alkoxy group or a benzyloxy group;

(2) 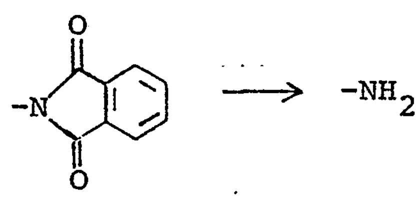

The reaction (1) or (2) may be effected by treating the amide, the urethane or the phthalimide under a basic or acidic aqueous medium.

Caustic alkalis such as sodium hydroxide, potassium hydroxide and barium hydroxide may preferably be used for these reactions, and hydrohalogenic acids such as hydrochloric acid, hydrobromic acid and hydroiodic acid may also preferably be used.

The reactions (1) and (2) may be carried out using a co-solvent such as methanol, ethanol, butanol, ethyleneglycol, dimethylformamide or dimethylsulfoxide. The reactions (1) and (2) may mostly be carried out by heating the

reaction mixture at a temperature from room temperature to the refluxing temperature of the reaction system in order to complete the hydrolysis.

The reaction (2) may also be achieved by the modified method of Manske (cf. J.Chem.Soc., 1926, 2348); namely, treatment of the phthalimide with hydrazine hydrate may afford the phthalhydrazide, and the resulting phthalhydrazide may easily be hydrolyzed by treatment with hydrochloric acid.

(Ad) via Rearrangement

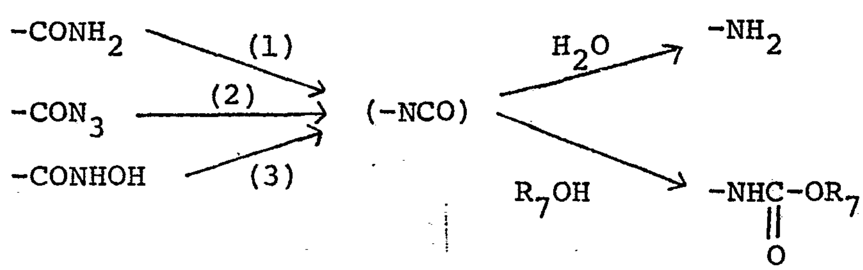


wherein $R_7$ is a $C_1$-$C_4$ alkyl group or a benzyl group.

The types of the reactions (1), (2) and (3) are all well known rearrangement reactions; the reaction (1) is Hofmann rearrangement (cf. Org. Reactions, 3, 267); the reaction (2) is Curtius rearrangement (cf. Org. Reactions, 3, 337); and the reaction (3) is Lossen rearrangement (cf. Chem. Review, 33, 209 (1943)).

By these reactions, the isocyanate may initially be formed, from which the amine may be obtained by hydrolysis, and the urethane may be obtained by treatment with an alcohol ($R_7$-OH).

The reaction (1) may be effected by treatment of the amide with an alkali hypohalite (e.g. sodium hypo-

bromite, sodium hypochlorite). The reaction (2) or (3) may be effected by heating the acyl azide or the hydroxamic acid. The reaction (3) may also be effected by treating the hydroxamic acid with a base (e.g. sodium hydride).

The reactions (1) to (3) may generally be carried out in a suitable solvent (e.g. water, methanol, ethanol, tetrahydrofuran, benzene, toluene) at a temperature from room temperature to the refluxing temperature of the reaction system.

The isocyanate obtained as above may be hydrolyzed into the amine in a conventional way. For example, the hydrolysis can be carried out by treating the isocyanate with an alkali (e.g. NaOH, KOH) or an acid (e.g. HCl, $H_2SO_4$) in water or a mixture of water and an organic solvent (e.g. dioxane, ethyleneglycol, dimethylformamide) at a temperature ranging from room temperature to the refluxing temperature of the reaction system.

When the reaction (1), (2) or (3) is carried out in the presence of an alcohol ($R_7$-OH), the urethane may be obtained as a product of the rearrangement.

The heterotricyclic cage compound thus obtained can be separated from the reaction mixture and purified by the conventional procedures.

Method B

The compound of the formula (If):

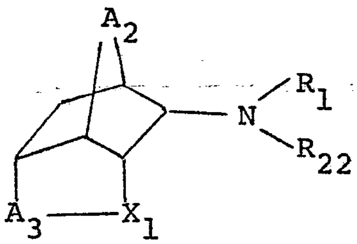

(If)

wherein $R_1$, $R_{22}$, $A_2$, $A_3$ and $X_1$ are each as defined above can be prepared from the tricyclic cage compound having the formula (IIb):

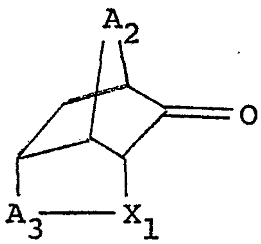

(IIb)

wherein $A_2$, $A_3$ and $X_1$ are each as defined above by the following method:

<u>(Ba) via Reduction</u>

(1) (IIb) 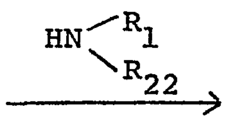 (If)

wherein $R_1$ and $R_{22}$ are each as defined above;

(2) (IIb) 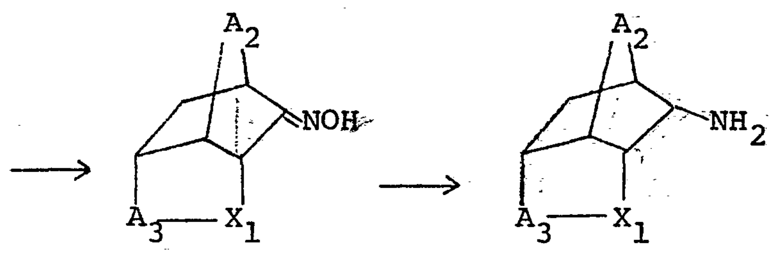

wherein $A_2$, $A_3$ and $X_1$ are each as defined above.

The reaction (1) is known as the reductive amination, and it may be effected by treating the carbonyl compound (IIb) with ammonia or an amine in the presence of a reducing agent such as a metal borohydride, an active metal

and an acid or formic acid or its derivative in a solvent (e.g. methanol, ethanol, benzene, toluene).

The reaction (2) is carried out by treating the carbonyl compound (IIb) with hydroxylamine, followed by reduction of the resulting oxime. This reduction may be preferably be carried out by treatment with a metal hydride compound such as lithium aluminum hydride, sodium tri-alkoxyaluminum hydride, sodium diethylaluminum hydride or sodium bis(methoxyethoxy)aluminum hydride in an inert solvent (e.g. ether, tetrahydrofuran, toluene).

The reduction in the reaction (1) or (2) may be also effected by hydrogenation in the presence of a catalyst such as palladium charcoal, platinum oxide or Raney nickel in an inert solvent (e.g. methanol, ethanol, benzene, toluene). The temperature for the above reduction may vary depending on the type of reduction or reducing agent to be used therein, but it may generally be from -20°C to the boiling point of the solvent to be used.

Method C

The compound of the formula (Ig):

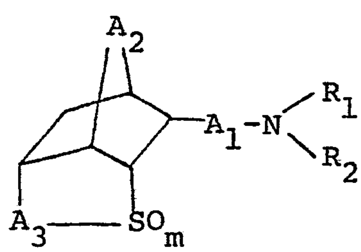

(Ig)

wherein $A_1$, $A_2$, $A_3$, $R_1$ and $R_2$ are each as defined above and m is an integer of 1 or 2 can be prepared by reacting the compound of the formula (Ih):

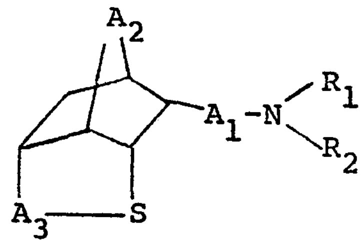

(Ih)

wherein $A_1$, $A_2$, $A_3$, $R_1$ and $R_2$ are each as defined above, with an oxidizing agent.

Examples of the oxidizing agent are hydrogen peroxide, peracids (e.g peracetic acid, perbenzoic acid, m-chloroperbenzoic acid), potassium permanganate, potassium persulfate, sodium hypochlorite, sodium metaperiodate, etc. The reaction may preferably be carried out in a solvent such as water, acetic acid, chloroform, dichloroethane, methanol, ethanol or benzene.

The oxidation may proceed in two steps: oxidation of the sulfide into the sulfoxide and that of the sulfoxide into the sulfone. The first step may proceed under milder conditions, e.g. at a low temperature from -50°C to room temperature or with a small amount of the oxidizing agent, so that the sulfoxide will substantially be obtained as a sole reaction product. The oxidation of the sulfide or the sulfoxide into the sulfone may preferably be effected by using an excess amount of the oxidizing agent under a relatively high temperature, i.e. from room temperature to the boiling point of the solvent.

The heterotricyclic cage compound thus obtained can be separated from the reaction mixture and purified by per se conventional procedures.

The key intermediates in the above Methods of this invention can be prepared by the following methods:

Method D

a) The compound of the formula (IIc) (the starting compound for Methods A and B):

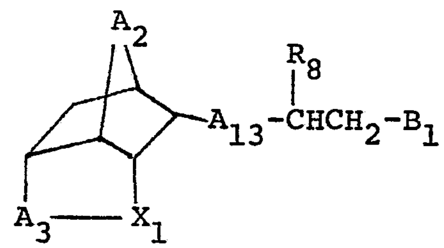

(IIc)

wherein $A_2$, $A_3$ and $X_1$ are each as defined above, $A_{13}$ is a single bond, a $C_2$-$C_3$ alkylene group or a $C_1$-$C_3$ alkylidene group, $R_8$ is a hydrogen atom, a methyl group or an ethyl group and $B_1$ is a carboxyl group, a $C_2$-$C_5$ alkoxycarbonyl group or a cyano group, can be prepared by hydrogenation of the compound of the formula (IId):

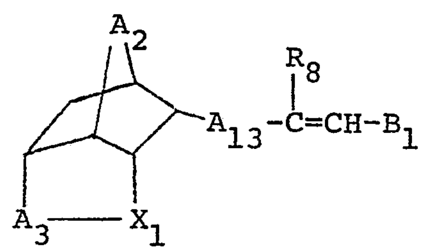

(IId)

wherein $A_2$, $A_3$, $X_1$, $A_{13}$, $R_8$ and $B_1$ are each as defined above.

b) The compound of the formula (IIe) (the starting compound for Methods A and B):

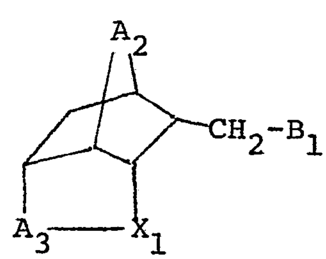

(IIe)

wherein $A_2$, $A_3$, $X_1$ and $B_1$ are each as defined above, can be prepared by hydrogenation of the formula (IIf):

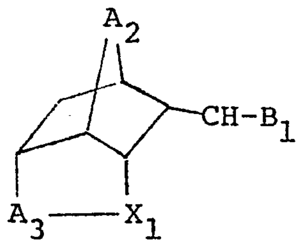

(IIf)

wherein $A_2$, $A_3$, $X_1$ and $B_1$ are each as defined above.

Hydrogenation may be carried out in the presence of a catalyst such as palladium on charcoal, platinum oxide or Raney nickel, preferably in an inert solvent (e.g. water, methanol, ethanol, toluene). The temperature for the hydrogenation may vary depending on the catalyst to be used and generally be from -20°C to the boiling point of the solvent to be used.

Method E

The compounds of the following formulae (the starting compounds for Method A):

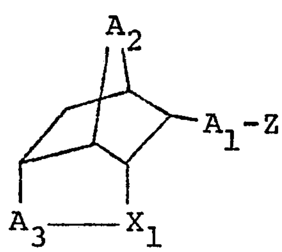

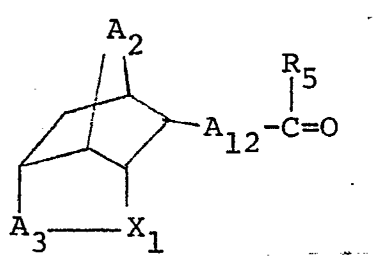

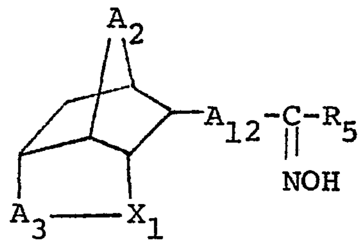

and

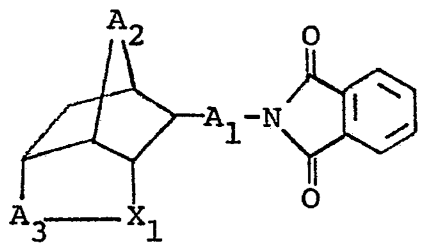

wherein $A_1$, $A_2$, $A_3$, $A_{12}$, $X_1$, Z and $R_5$ are each as defined above, can be prepared by introduction of the functional groups in the corresponding compounds having no such functional groups according to per se conventional procedures.

Typical examples are shown in the following scheme:

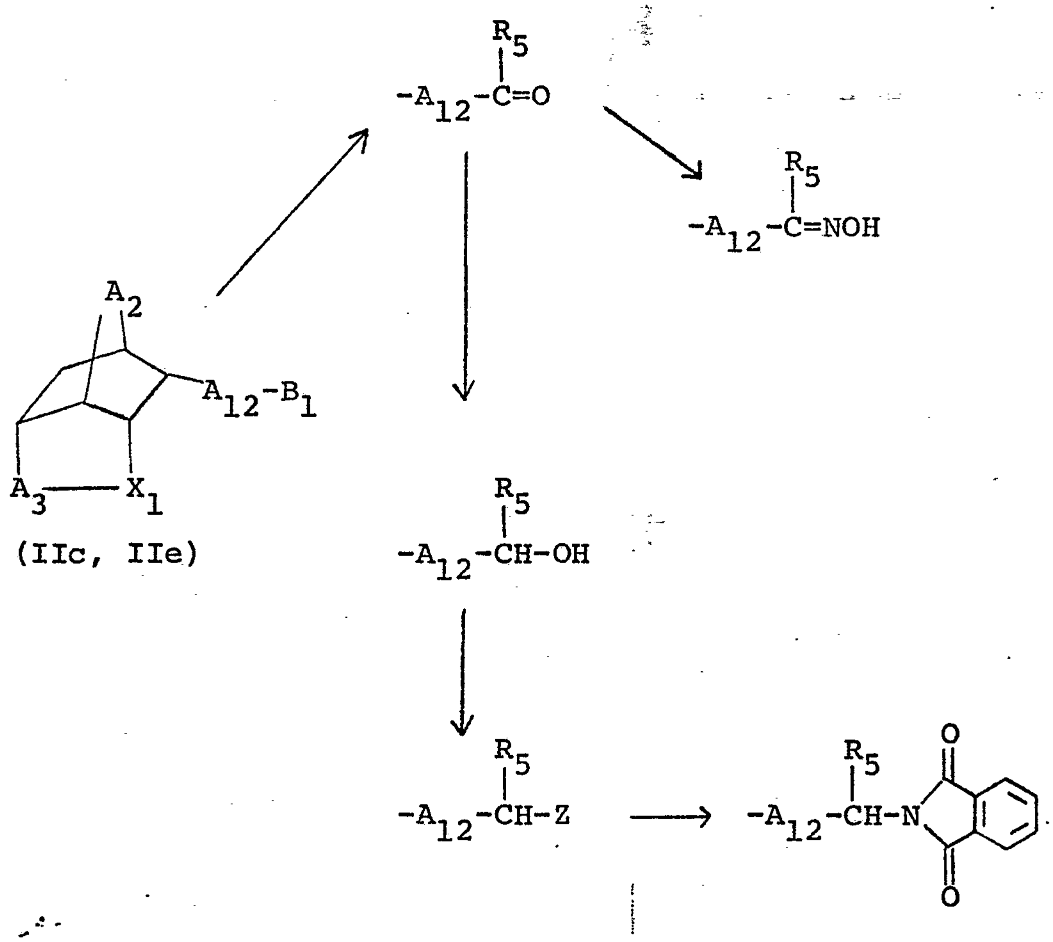

wherein $A_2$, $A_3$, $A_{12}$, $X_1$, Z, $R_5$ and $B_1$ are each as defined above.

Method F

The compounds of the following formulae (IIb), (IIg) and (IIh) (the starting compounds for Methods A and B) can be prepared by the following chemical conversions:

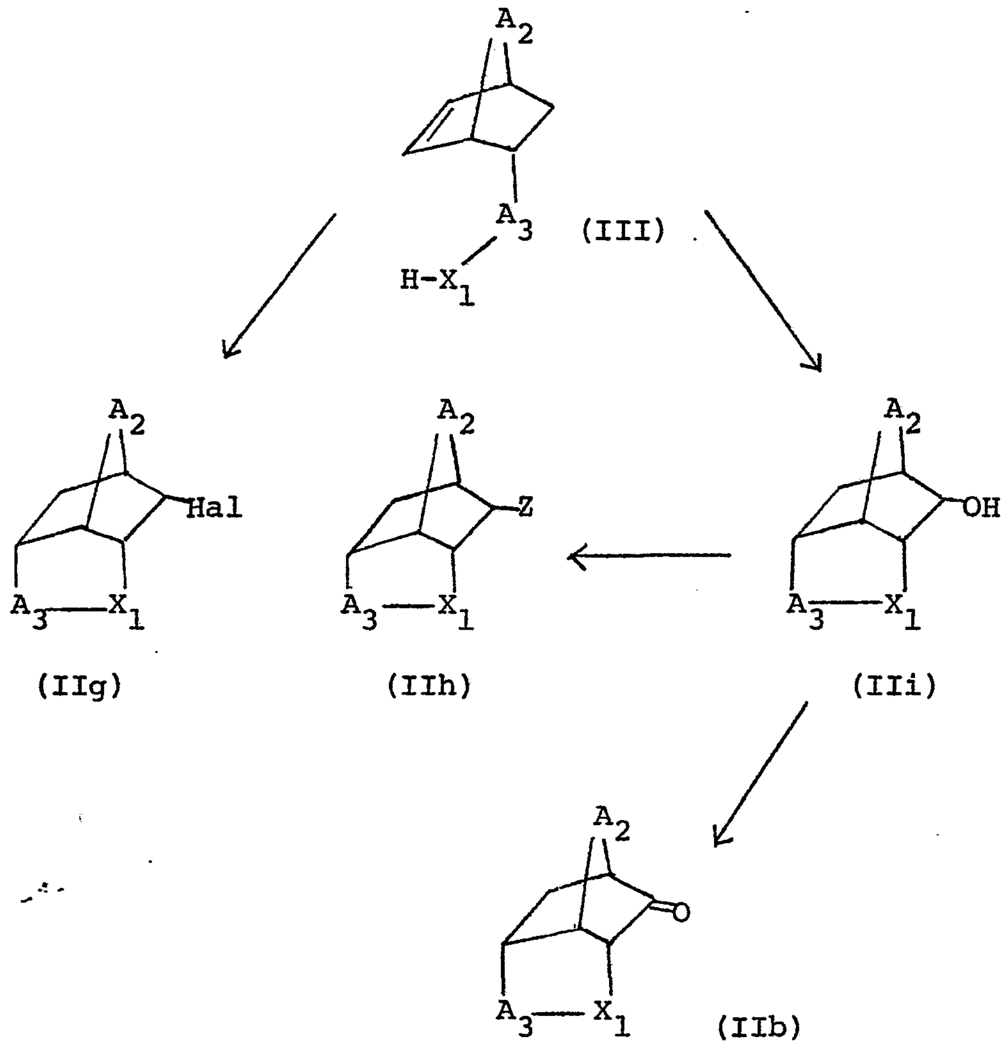

(III)

(IIg) (IIh) (IIi)

(IIb)

wherein $A_2$, $A_3$, $X_1$ and Z are each as defined above and Hal is halogen.

The compound (IIg) can be prepared by reacting the compound (III) with a halogen compound. Examples of the halogen compound are bromine, iodine, N-bromosuccinic imide (NBS) and so on. The reaction may be preferably be carried out in an inert solvent (e.g. water, carbon tetrachloride, chloroform, dichloroethane, tetrahydrofuran). The reaction proceeds generally at a temperature from 0°C to room temperature.

The compound (IIi) can be prepared by oxidative cyclization of the compound (III); e.g. treatment of the compound (III) with an oxidizing agent such as lead tetraacetate or mercuric salts (e.g. $Hg(OCOCH_3)_2$, $Hg(OCOCCl_3)_2$, $Hg(OCOCF_3)_2$, $Hg(NO_3)_2$), followed by oxidizing the resulting product with an ozone. The reaction may be carried out in the presence of an inert solvent (e.g. water, acetic acid, benzene, tetrahydrofuran, dioxane, dimethylformamide).

Conversion of the compound (IIi) into the compound (IIh) may be carried out in a per se conventional manner. That is, the compound (IIh) can be prepared by reacting the compound (IIi) with thionyl chloride, phosphorus halide, phosphorous oxyhalide, p-toluenesulfonyl chloride, methane-sulfonylchloride or the like. Examples of the residue of a strong acid represented by Z are chloride (-Cl), bromide (-Br), p-toluenesulfonate ($-OSO_2-$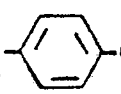$-CH_3$), methanesulfonate ($-OSO_2-CH_3$), sulfate ($-O-SO_2-O-$) and so on. The reaction may preferably be carried out in the presence of an organic or inorganic base.

The compound (IIb) can be prepared by reacting the compound (IIi) with an oxidizing agent. Examples of the oxidizing agent are pyridinium chlorochromate, potassium permanganate, chromium trioxide, etc. The reaction may preferably be carried out in a solvent such as water, acetic acid, chloroform, dichloroehtane or benzene.

Method G

The compounds of the formulae (IId) and (IIf),

which are the starting compounds for Method D, can be prepared by reacting the carbonyl compound shown below with the Witting reagent of the formula: $(C_6H_5)_3P=CH-B_1$ or $(R_3O)_2\overset{O}{\overset{\uparrow}{P}}CH-B_1$ (wherein $R_3$ and $B_1$ are each as defined above). Namely, the following chemical conversions are effected:

(a) 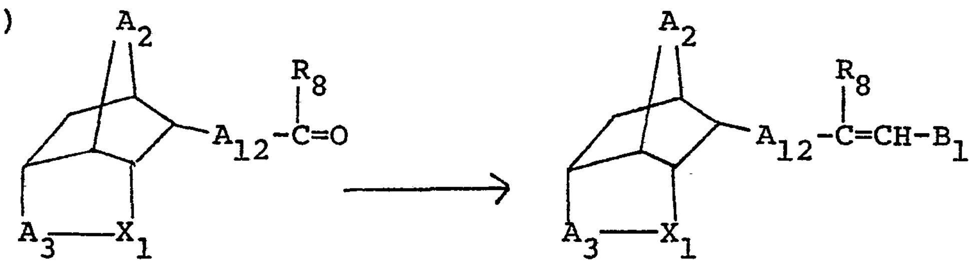

(IIj) (IId)

wherein $A_2$, $A_3$, $A_{12}$, $X_1$, $R_8$ and $B_1$ are each as defined above; and

(b) 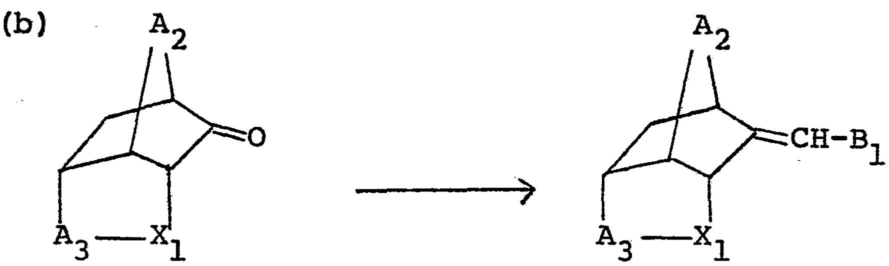

(IIb) (IIf)

wherein $A_2$, $A_3$, $X_1$ and $B_1$ are each as defined above.

The reaction may be carried out in an inert solvent such as benzene, ether, tetrahydrofuran, methylene chloride, dimethylformamide, dimethylsulfoxide, alcohol or water at a temperature ranging from 0°C to the boiling point of the solvent.

Method H

The key intermediate compounds of the following formulae (IIk), (IIl), (IIm), (IIn), (IIo), (IIp) and (IIq) can be prepared from the compound (IIe) according to the following conversions:

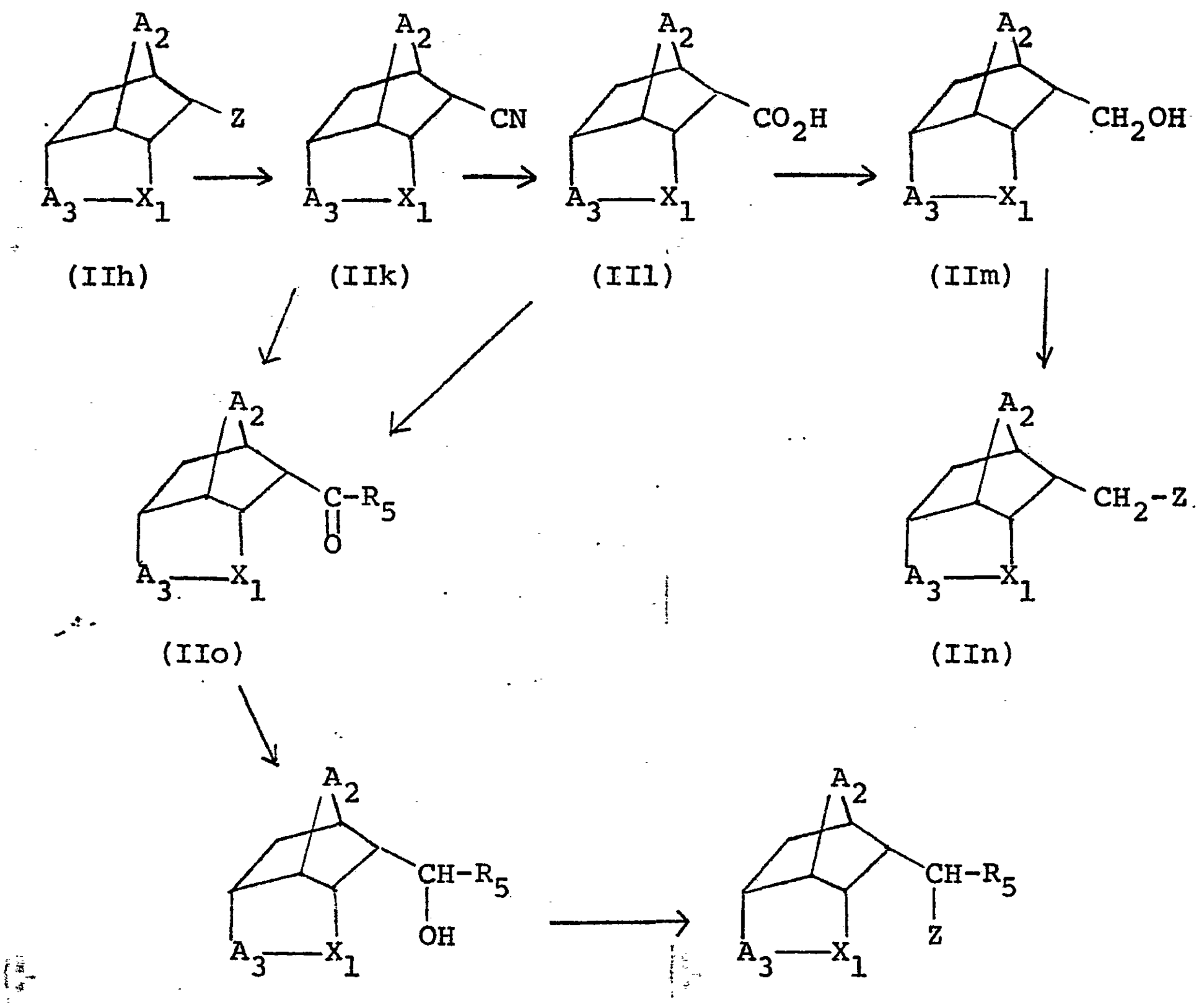

(IIh) (IIk) (IIl) (IIm) (IIo) (IIn) (IIp) (IIq)

wherein $A_2$, $A_3$, $X_1$, Z and $R_5$ are each as defined above.

Conversion of the compound (IIh) into the compound (IIm) may be carried out in a per se conventional manner. That is, the compound (IIm) can be prepared by reacting the compound (IIh) with alkali metal cyanide, hydrolyzing the resulting compound (IIk) and reducing the resultant compound

(IIl) in its ester form with metal hydride. The compound (IIo) can be prepared by reacting the compound (IIk) or (IIl) with $R_5Li$, $R_5MgBr$ or $(R_5)_2Cd$ and converted into the compound (IIp) by reducing with metal hydride or hydrogenation. The compounds (IIn) and (IIq) are obtainable by reacting the compounds (IIm) or (IIp) respectively with thionyl chloride, p-toluenesulfonyl chloride, methanesulfonyl chloride or the like.

The compound of the formula (III), i.e. the common intermediate for the synthesis of the compound (I) of the present invention:

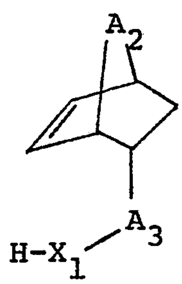

(III)

wherein $A_2$, $A_3$ and $X_1$ are each as defined above, can be prepared from an alicyclic compound by the Diels-Alder reaction, optionally followed by appropriate functional modifications.

[Step 1] The Diels-Alder reaction

The first step for the synthesis of the compound (III) is the Diels-Alder reaction using an alicyclic diene compound of the formula (IVa):

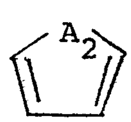

(IVa)

wherein $A_2$ is as defined above with a dienophile of the formula (IVb):

$CH_2=CH-A_4-V$ (IVb)

wherein $A_4$ is a single bond or a lower alkylene group or a lower alkylidene group and V is a carboxyl group, an alkoxycarbonyl group, a cyano group, a formyl group or an acyl group. The resulting product is the diene adduct of the formula:

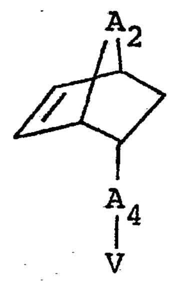

(IIIa)

wherein $A_2$, $A_4$ and V are each as defined above.

The above reaction may be carried out in a conventional manner as disclosed, for example, in H. Wollweber in "Methoden der Organischen Chemie (Houben-Wyle)", Band V/IC, Kohlenwasserstoffe III, Georg Thieme (1970), 977-1139; M.P. Kloetzel: Org. React., 4, 1-59 (1948); H.L. Holmes: ibid., 4, 60-173 (1948); L.W. Butz, A.W. Rytina: ibid., 5, 136-192 (1949). The diene adduct (IIIa) thus prepared can be separated from the reaction mixture by a conventional procedure such as chromatography or recrystallization.

[Step 2] Functional modification

The second step is the transformation of the group ($-A_4-V$) with the endo-orientation of the diene adduct obtained above into the group ($-A_3-X_1-H$) as shown below:

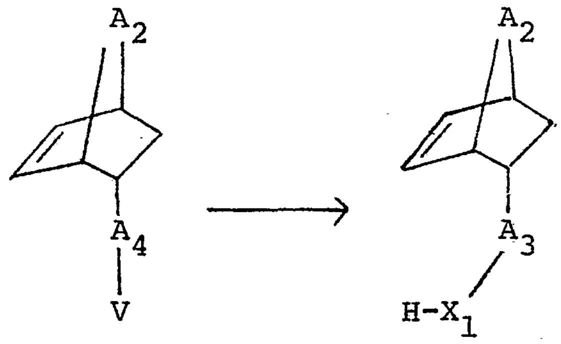

(IIIa) (III)

The modification of the functional group of the diene adducts may be carried out by using a conventional procedure such as hydrolysis, reduction or thiolation. For example, the compounds (III) wherein $-A_3-X_1-H$ is hydroxymethyl, β-hydroxyethyl, thiolmethyl or carboxy may be obtained from the corresponding compounds (IIIa) wherein $-A_4-V$ is alkoxycarbonyl, alkoxycarbonylmethyl, halomethyl by reduction, thiolation or hydrolysis.

The alicyclic diene compound (IVa) may be, for instance, cyclopentadiene, 1,3-cyclohexadiene, cycloheptatriene or the like.

The followings are typical examples of the diene adducts to be used in the production of the compound (III):

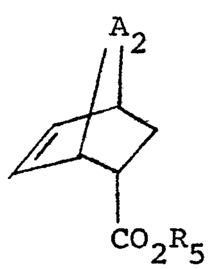

(1)

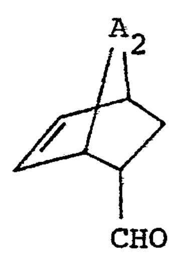

(2)

wherein $A_2$ and $R_5$ are each as defined above.

Among the diene adducts, those of the formulae

(1) ($A_1$ = $CH_2$, $CH_2CH_2$) and (2) ($A_1$ = $CH_2$, $CH_2CH_2$) are already known.

Some of the typical transformation starting from the diene adduct into the objective compound (I) of the present invention are illustrated in Chart A.

<u>Chart A</u>

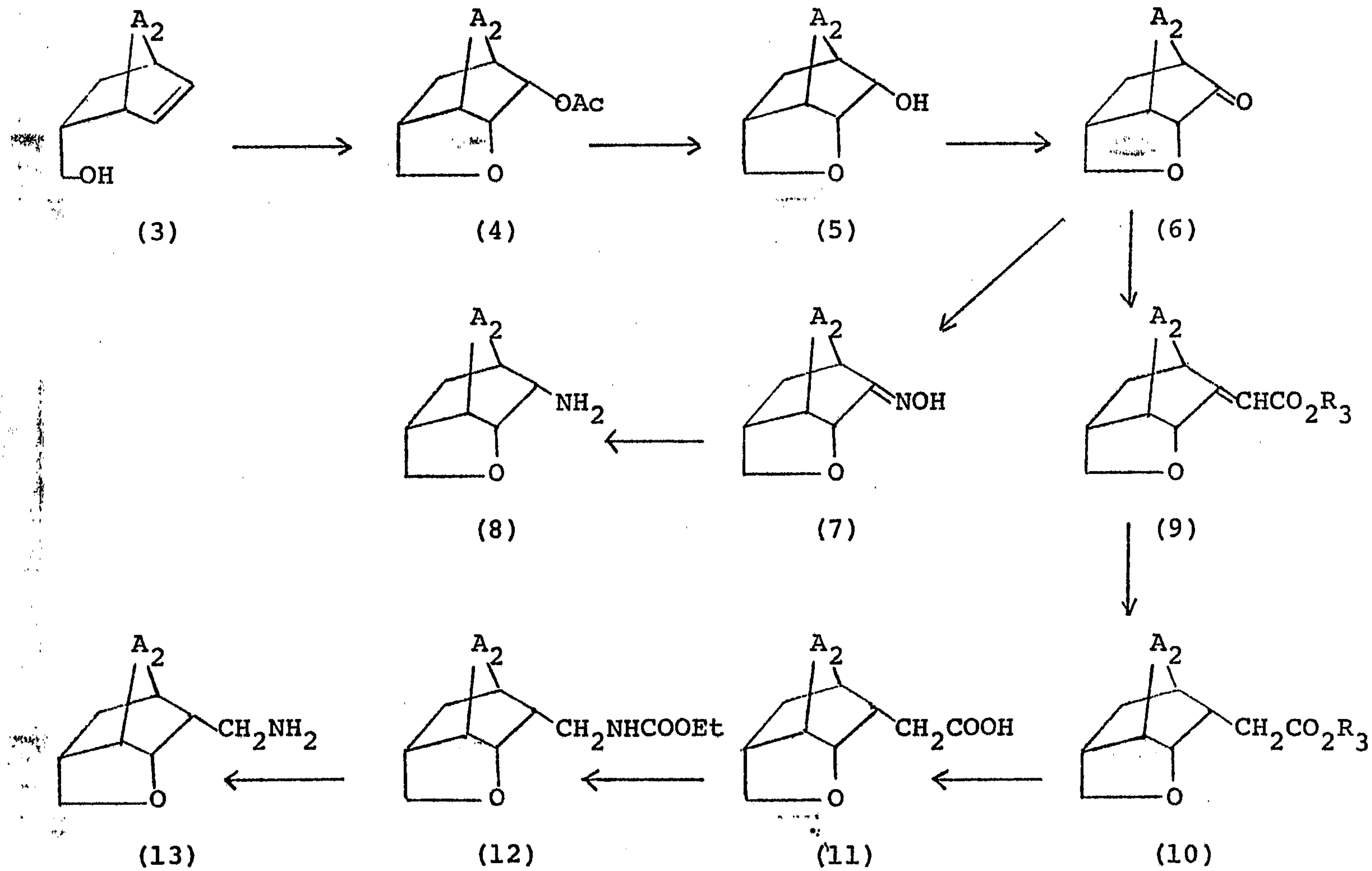

(3) (4) (5) (6) (7) (8) (9) (10) (11) (12) (13)

wherein $A_2$ and $R_3$ are each as defined above.

The compound (4: $A_2 = CH_2CH_2$) can be obtained from the compound (3) by oxy-acetoxylation:

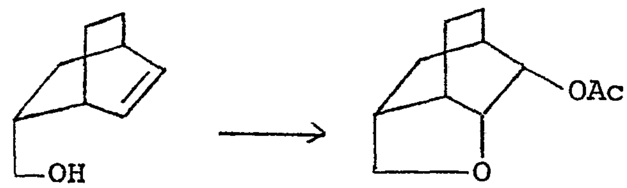

(Preparative Example 5)

Hydrolysis of the compound (4: $A_2 = CH_2$, $CH_2CH_2$) gives the alcohol compound (5):

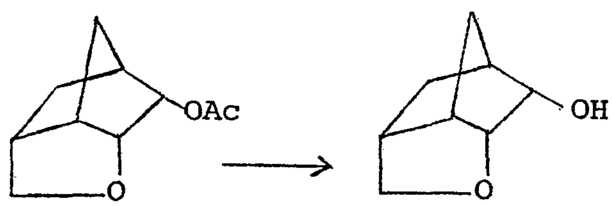

(Preparative Example 1)

Tetrahedron Letters, 1165 (1964)

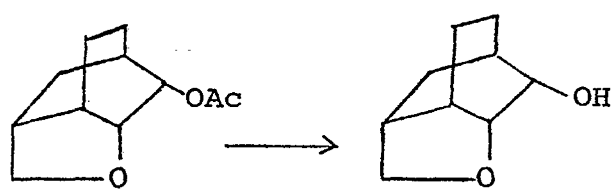

(Preparative Example 6)

Oxidation of the compound (5) with dimethylsulfoxide-dicyclohexylcarbodiimide gives the ketone (6): Preparative Example 2, Preparative Example 7.

The compound (8) are obtained by reduction of the compound (7) with lithium aluminum hydride: Example 1, Example 5.

a-2

The Wittig reaction of the compound (6) gives the olefinic ester (9): Preparative Example 9.

The hydrogenation of the compound (9) gives the

compound (10): Preparative Example 10.

The compound (13) can be achieved by a sequence of hydrolysis, Curtius reaction and hydrolysis from the compound (10): Preparative Example 11, Example 6, Example 7.

As to the production of the compound (I) of the invention, some typical procedures have hereinabove illustrated in detail. However, their production procedures are not restricted to them. Some typical embodiments are shown in Examples and Preparative Examples, which are summarized in the following scheme:

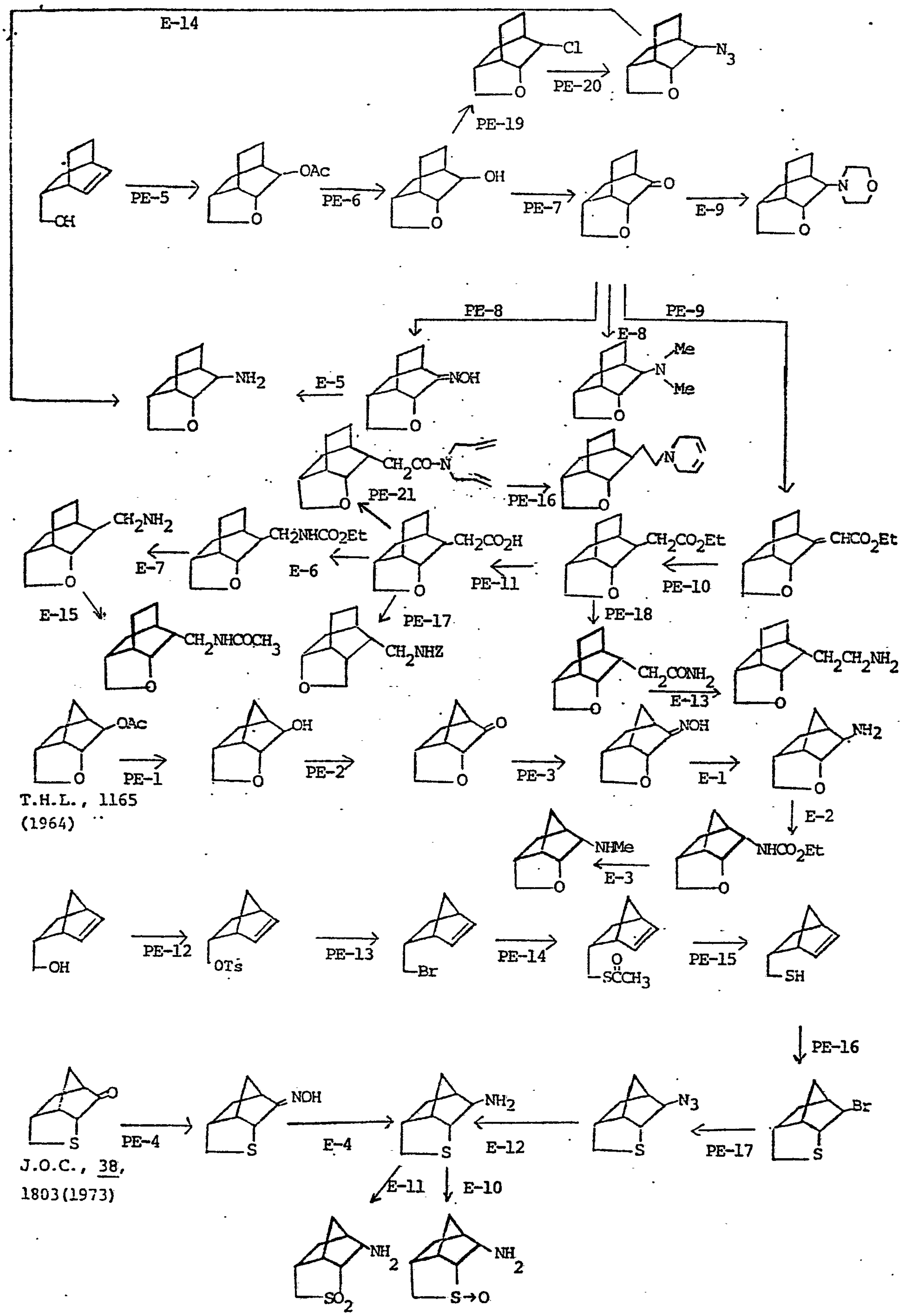
E-14
PE-20
PE-19
PE-5
PE-6
PE-7
E-9
PE-8
E-8
PE-9
E-5
PE-21
PE-16
E-7
E-6
PE-11
PE-10
E-15
PE-17
PE-18
E-13
PE-1
PE-2
PE-3
E-1
T.H.L., 1165
(1964)
E-2
E-3
PE-12
PE-13
PE-14
PE-15
PE-16
PE-4
E-4
E-12
PE-17
J.O.C., 38,
1803(1973)
E-11
E-10

Practical and presently preferred embodiments of the invention are illustratively shown in the following examples, to which the present invention will be in no way limited.

Preparative Example 1

4-Oxa-2-acetoxy-brendane [Tetrahedron Letters, 1165 (1964)] (15 g) was dissolved in a mixed solvent of 150 ml of 10 % aqueous sodium hydroxide solution, 150 ml of tetrahydrofuran and 150 ml of methanol, and the resultant mixture was stirred at room temperature for 2 hours. After removal of tetrahydrofuran and methanol therefrom, the aqueous solution was extracted with ether. The ether extract was dried, concentrated and purified by silica gel column chromatography. From the eluate with benzene, there were obtained 11.8 g of 4-oxa-2-hydroxy-brendane as an oily substance. $IR\nu_{max}^{film}$ ($cm^{-1}$): 3300, 2950, 2850, 1240, 1080.

Preparative Example 2

A mixture of 4-oxa-2-hydroxy-brendane (3.3 g) and pyridinium chlorochromate (10.24 g) in 100 ml of methylene chloride was stirred at room temperature for 1 hour. After removal of methylene chloride, the reaction mixture was extracted with ether. Concentration of the ether extract and purification of the residue by silica gel column chromatography using benzene as an eluent gave 1.7 g of 4-oxa-2-oxo-brendane as an oily substance. $IR\nu_{max}^{film}$ ($cm^{-1}$): 2950, 2850, 1750, 1060.

Preparative Example 3

To a mixture of 4-oxa-2-oxo-brendane (1.7 g), hydroxylamine hydrochloride (1.4 g), ethanol (30 ml) and water (11 ml), sodium carbonate (5.7 g) was added, and the resultant mixture was refluxed for 4 hours. After cooling, water was added to the reaction mixture, which was then extracted with ethyl acetate. Drying and concentration of the extract gave 1.2 g of 4-oxa-2-hydroxyimino-brendane as an oily substance. $IR\nu_{max}^{film}$ ($cm^{-1}$): 3300, 2950, 2870, 1240, 1060.

Example 1

A solution of 4-oxa-2-hydroxyimino-brendane (1.2 g) in dry tetrahydrofuran (20 ml) was added to a mixture of lithium aluminum hydride (350 mg) and tetrahydrofuran (20 ml) with refluxing for 2 hours. After decomposition of the excessive lithium aluminum hydride with water, tetrahydrofuran was removed by distillation from the reaction mixture. The residue was extracted with a mixture of water and chloroform. The chloroform layer was separated, dried and concentrated to give 0.8 g of 4-oxa-2-amino-brendane as an oily substance. $IR\nu_{max}^{film}$ ($cm^{-1}$): 3380, 3300, 2950, 2870, 1600, 1360.

Example 2

4-Oxa-2-amino-brendane (1.2 g) as obtained in Example 1 and triethylamine (2.0 g) were dissolved in ether, and ethyl chlorocarbonate (0.9 g) was dropwise added thereto at room temperature. After completion of the

addition, the mixture was stirred at the same temperature as above for 3 hours and extracted with a mixture of water and ether. The ether layer was dried, concentrated and purified by silica gel column chromatography. From the eluent with chloroform, there was obtained 0.5 g of 4-oxa-4-ethoxycarbonylamino-brendane as an oily substance. $IR\nu_{max}^{film}$ ($cm^{-1}$): 3420, 2950, 2870, 1700, 1500, 1310, 1080.

Example 3

4-Oxa-2-ethoxycarbonylamino-brendane (0.5 g) and lithium aluminum hydride (0.2 g) were refluxed in tetrahydrofuran for 8 hours. Working up in the same manner as in Example 1 gave 0.2 g of 4-oxa-2-methylamino-brendane as an oily substance. $IR\nu_{max}^{film}$ ($cm^{-1}$): 3310, 2950, 2860, 2790, 1600, 1500, 1050.

Preparative Example 4

In the same manner as in Preparative Example 3, 4-thia-2-oxo-brendane [J.O.C., 38, 1803 (1973)] (10 g) gave 9.2 g of 4-thia-2-hydroxyimino-brendane as an oily substance. $IR\nu_{max}^{film}$ ($cm^{-1}$): 3200 - 3100, 1690, 1620, 1580.

Example 4

In the same manner as in Example 1, 4-thia-2-hydroxyimino-brendane (6 g) gave 2.0 g of 4-thia-2-amino-brendane as an oily substance. $IR\nu_{max}^{film}$ ($cm^{-1}$): 3300, 3250, 2950, 2870, 1580.

Preparative Example 5

2-Hydroxymethylbicyclo[2,2,2]oct-5-ene (14.2 g), lead tetraacetate (44 g) and calcium carbonate (5 g) were

added to 250 ml of benzene, and the resultant mixture was refluxed for 7 hours. After filtration, the oily substance was purified by silica gel column chromatography using benzene as an eluent. This gave 11.6 g of 4-oxa-2-acetoxy-isotwistane as an oily substance. $IR\nu_{max}^{film}$ ($cm^{-1}$): 2950, 2870, 1735, 1370, 1250, 1220, 1100, 1025, 1000.

Preparative Example 6

In the same manner as in Preparative Example 1, 4-oxa-2-acetoxy-isotwistane (11.6 g) gave 6.5 g of 4-oxa-2-hydroxy-isotwistane as an oily substance.

Preparative Example 7

In the same manner as in Preparative Example 2, 4-oxa-2-hydroxy-isotwistane (1 g) gave 600 mg of 4-oxa-2-oxo-isotwistane as an oily substance. $IR\nu_{max}^{film}$ ($cm^{-1}$): 2950, 2870, 1730, 1465, 1380.

Preparative Example 8

In the same manner as in Preparative Example 3, 4-oxa-2-oxo-isotwistane (600 mg) gave 230 mg of 4-oxa-2-hydroxyimino-isotwistane as crystals. M.P., 107 - 109°C. $IR\nu_{max}^{film}$ ($cm^{-1}$): 3220, 2930, 2860, 1085, 980, 910.

Example 5

In the same manner as in Example 1, 4-oxa-2-hydroxyimino-isotwistane (500 mg) gave 300 mg of 4-oxa-2-amino-isotwistane as an oily substance. $IR\nu_{max}^{film}$ ($cm^{-1}$): 3370, 3300, 2930, 2870, 1480, 1070, 680.

Preparative Example 9

A solution of ethoxycarbonylmethyl diethylphos-

phonate (2.65 g) in 5 ml of tetrahydrofuran was dropwise added to a solution of sodium hydride (0.44 g) in 15 ml of tetrahydrofuran. After completion of the addition, stirring was continued until the generation of hydrogen gas stopped. A solution of 4-oxa-2-oxo-isotwistane (0.90 g) obtained in Preparative Example 7 in 5 ml of tetrahydrofuran was added thereto, and stirring was effected at room temperature for 2 hours. After addition of water, the reaction mixture was extracted with ethyl acetate, and the extract was dried and concentrated. Purification of the residue by silica gel column chromatography using benzene/ethyl acetate as an eluent gave 0.86 g of 4-oxa-2-ethoxycarbonylmethylidene-isotwistane as an oily substance. IR$\nu_{max}^{film}$ ($cm^{-1}$): 2940, 2860, 1715, 1650, 1480, 1450, 1380, 1240, 1200.

Preparative Example 10

4-Oxa-2-ethoxycarbonylmethylidene-isotwistane (0.86 g) was dissolved in 20 ml of ethanol. After addition of 0.2 g of a 10 % palladium carbon, the resultant mixture was stirred under hydrogen stream for 5 hours and filtered. The filtrate was subjected to purification by silica gel column chromatography using benzene/ethyl acetate as an eluent to give 0.78 g of 4-oxa-2-ethoxycarbonylmethyl-isotwistane as an oily substance. IR$\nu_{max}^{film}$ ($cm^{-1}$): 2930, 2860, 1730, 1300, 1150, 1070, 1030.

Preparative Example 11

4-Oxa-2-ethoxycarbonylmethyl-isotwistane (0.62 g) was dissolved in 5 ml of methanol, and sodium hydroxide (1

g) and water (10 ml) were added thereto. The resultant mixture was stirred overnight. The neutral portion was extracted with chloroform, made acidic with potassium hydrogensulfate and extracted with ethyl acetate. The extract was dried and concentrated to give 0.44 g of 4-oxa-2-carboxymethyl-isotwistane. IR$\nu_{max}^{film}$ ($cm^{-1}$): 2930, 2870, 2650, 1700, 1410, 1240.

Example 6

4-Oxa-2-carboxymethyl-isotwistane (0.43 g) was dissolved in 8 ml of acetone, followed by addition of triethylamine (288 mg) thereto. To the resultant mixture cooled to -20°C, ethyl chlorocarbonate (285 mg) was added, and the reaction mixture was stirred at the same temperature as above for 1 hour. The mixture was further cooled to -30°C, a solution of sodium azide (285 mg) in 5 ml of water was added thereto, and stirring was continued for 1 hour. The reaction mixture was allowed to warm up to room temperature and extracted with benzene. The extract was dried, concentrated and refluxed for 2 hours with addition of ethanol. The mixture was concentrated and subjected to purification by silica gel column chromatography to give 0.5 g of 4-oxa-2-ethoxycarbonylaminomethyl-isotwistane as an oily substance. IR$\nu_{max}^{film}$ ($cm^{-1}$): 3320, 2930, 2870, 1700, 1540, 1260, 1080, 1040.

Example 7

A mixture of 4-oxa-2-ethoxycarbonylaminomethyl-isotwistane (0.5 g), potassium hydroxide (0.6 g) and

ethylene glycol (2 ml) was refluxed for 15 minutes. After cooling, the reaction mixture was added with water and extracted with chloroform. The extract was washed with water, dried and concentrated to give 0.34 g of 4-oxa-2-aminomethyl-isotwistane as an oily substance. $IR\nu_{max}^{film}$ ($cm^{-1}$): 3350, 3290, 2910, 2850, 1080.

Example 8

To a solution of dimethylamine hydrochloride (1.0 g) in 10 ml of methanol, potassium hydroxide (0.2 g) was added, and the resultant mixture was stirred at room temperature for 30 minutes. 4-Oxa-2-oxo-isotwistane (500 mg) obtained in Preparative Example 7 was added thereto and stirred at room temperature for 15 minutes. To the resultant mixture, sodium cyanoborohydride (250 mg) was added, and the resulting mixture was stirred at room temperature for 2 hours. After removal of methanol by distillation, the residue was extracted with benzene, and the extract was dried and concentrated to give 0.2 g of 4-oxa-2-N,N-dimethylamino-isotwistane as an oily substance. $IR\nu_{max}^{film}$ ($cm^{-1}$): 2950, 2860, 1460, 1060.

Example 9

In the same manner as in Example 8 but using morpholine in place of dimethylamine, there was produced 4-oxa-2-N-morpholino-isotwistane as an oily substance. $IR\nu_{max}^{film}$ ($cm^{-1}$): 2950, 1430, 1050.

Example 10

4-Thia-2-amino-brendane (300 mg) obtained in

Example 4 and potassium periodate (280 mg) were added to a mixture of 15 ml of water and 15 ml of methanol, and the resultant mixture was stirred at room temperature for 1 hour. After removal of methanol by distillation, the residue was extracted with chloroform. The extract was washed, dried and concentrated to give 100 mg of 4-thia-4-oxo-2-amino-brendane as an oily substance. IR$\nu_{max}^{film}$ ($cm^{-1}$): 3300, 3250, 2950, 1450, 1040, 1020.

Example 11

4-Thia-2-amino-brendane (200 mg) obtained in Example 4 was dissolved in 6 ml of 8N acetic acid, a solution of potassium permanganate (0.3 g) in water (4 ml) was added thereto, and the mixture was stirred at 25°C for 30 minutes. The reaction mixture was cooled with ice-water and bubbled with sulfur dioxide gas until the mixture discolored. The mixture was then adjusted to pH 12 and extracted with chloroform. The extract was washed, dried and concentrated to give 50 mg of 4-thia-4,4-dioxo-2-amino-brendane as an oily substance. IR$\nu_{max}^{film}$ ($cm^{-1}$): 3300, 3230, 2950, 1280, 1110.

Preparative Example 12

To a mixture of commercially available endo-2-hydroxymethyl-5-norbornene (31 g) and p-toluenesulfonyl chloride (60 g) in 1000 ml of benzene, 200 ml of pyridine were dropwise added while cooling with ice-water. After stirring overnight, the reaction mixture was made acidic with hydrochloric acid and extracted with benzene. The

extract was successively washed with dilute hydrochloric acid and an aqueous sodium bicarbonate solution, and dried. Concentration of the dried extract gave 80 g of endo-2-p-toluenesulfonyloxymethyl-5-norbornene as a solid substance. $IR\nu_{max}^{film}$ ($cm^{-1}$): 1600, 950, 870.

Preparative Example 13

A mixture of endo-2-p-toluenesulfonyloxymethyl-5-norbornene (80 g) obtained in Preparative Example 12 and lithium bromide (250 g) in 500 ml of methyl ethyl ketone was refluxed for 3 hours. The reaction mixture was concentrated and extracted with water/chloroform. The chloroform layer was washed, dried and concentrated. Distillation of the concentrate gave 50 g of endo-2-bromomethyl-5-norbornene as an oily substance boiling at 90°C/30 mmHg. $IR\nu_{max}^{film}$ ($cm^{-1}$): 3050, 2950, 1450, 1430, 1330, 1250, 1220.

Preparative Example 14

A solution of endo-2-bromomethyl-4-norbornene (5 g) obtained in Preparative Example 13 and potassium thioacetate (10 g) in a mixed solvent of 20 ml of dimethylformamide and 20 ml of dimethylsulfoxide was stirred at room temperature for 4 hours. The reaction mixture was poured into water and extracted with ether. The ether extract was washed, dried and concentrated to give 4.8 g of endo-2-acetylthiomethyl-5-norbornene as an oily substance. $IR\nu_{max}^{film}$ ($cm^{-1}$): 1700, 1350, 1340, 1140, 960.

Preparative Example 15

To a solution of endo-2-acetylthiomethyl-5-norbornene (4.8 g) obtained in Preparative Example 14 in dry methanol (100 ml), potassium carbonate (0.5 g) was added, and the resultant mixture was stirred at room temperature for 5 hours. After concentration, the mixture was extracted with water/chloroform to give 3.5 g of endo-2-mercaptomethyl-5-norbornene as an oily substance. $IR\nu_{max}^{film}$ ($cm^{-1}$): 3000, 1340, 1220.

Preparative Example 16

To a mixture of endo-2-mercaptomethyl-5-norbornene (1 g) obtained in Preparative Example 15 in 20 ml of anhydrous methylene chloride, bromine (2.3 g) was added at 0 - 5°C, and the resultant mixture was stirred for 10 minutes. After addition of sodium thiosulfate to decompose bromine, the reaction mixture was extracted with methylene chloride. The extract was washed, dried and concentrated to give 1.1 g of exo-2-bromo-4-thia-brendane as an oily substance. $IR\nu_{max}^{film}$ ($cm^{-1}$): 2950, 2870, 1450, 1230.

Preparative Example 17

A mixture of exo-2-bromo-4-thia-brendane (150 mg) obtained in Preparative Example 16 and sodium azide (200 mg) in 95 % aqueous ethanol (2 ml) was refluxed for 7 hours. The reaction mixture was concentrated and extracted with ether, and the extract was washed and dried. Concentration of the extract gave 100 mg of 2-azido-4-thia-brendane as an oily substance. $IR\nu_{max}^{film}$ ($cm^{-1}$): 2950, 2850, 2100, 1480, 1440, 1320, 1240, 680.

Example 12

A mixture of 2-azido-4-thia-brendane (100 mg) obtained in Preparative Example 17 and lithium aluminum hydride (0.5 g) in ether, was refluxed for 3 hours. After decomposition of the excessive lithium aluminum hydride with addition of water, the reaction mixture was extracted with ether. The extract was washed, dried and concentrated to give 90 mg of 4-thia-2-amino-brendane. The IR spectrum of this product was identical with that of the product obtained in Example 4.

Preparative Example 18

4-Oxa-2-ethoxycarbonylmethyl-isotwistane (0.5 g) obtained in Preparative Example 10 was added to 50 ml of conc. aqueous ammonia solution. The resultant mixture was stirred at room temperature for 5 days and extracted with chloroform. The chloroform extract was washed, dried, concentrated and subjected to purification by silica gel column chromatography using chloroform as an eluent to give 0.23 g of 4-oxa-2-carbamoylmethyl-isotwistane as a solid substance. $IR\nu_{max}^{film}$ ($cm^{-1}$): 3400, 3350, 1680, 1180, 1080.

Example 13

In the same manner as in Example 3, 4-oxa-2-carbamoylmethyl-isotwistane gave 4-oxa-2-β-aminoethyl-isotwistane as an oily substance. $IR\nu_{max}^{film}$ ($cm^{-1}$): 3330, 3290, 1180, 1080.

Preparative Example 19

4-Oxo-2-hydroxy-isotwistane (3 g) obtained in Preparative Example 6 was added to a mixture of pyridine

(2.5 g) and methylene chloride (25 ml). Under ice-cooling, thionyl chloride (3.8 g) was added thereto, and the resultant mixture was stirred at room temperature for 5 hours. After concentration of the reaction mixture, aqueous sodium bicarbonate solution was added to neutralize the mixture, which was then extracted with chloroform. The chloroform extract was dried and concentrated. Purification of the residue by silica gel column chromatography using benzene as an eluent gave 1.5 g of 4-oxo-2-chloro-isotwistane as an oily substance. $IR\nu_{max}^{film}$ ($cm^{-1}$): 2930, 2870, 1080.

Preparative Example 20

A mixture of 4-oxo-2-chloro-isotwistane (240 mg) and sodium azide (323 mg) in dimethylformamide (3 ml) was heated at 150°C for 2 hours. After cooling, the mixture was added with water and extracted with ether. The ether extract was dried and concentrated to give 180 mg of 4-oxo-2-azido-isotwistane as an oily substance. $IR\nu_{max}^{film}$ ($cm^{-1}$): 2950, 2870, 2100, 1450, 1280.

Example 14

In the same manner as in Example 12, 4-oxo-2-azido-isotwistane gave 4-oxo-2-amino-isotwistane. The IR spectrum of this product was identical with that of the product obtained in Example 5.

Example 15

To a mixture of 4-oxa-2-aminomethyl-isotwistane (200 mg) obtained in Example 7 and triethylamine (200 mg) in 5 ml of ether, acetyl chloride (100 mg) was dropwise

added. After stirring at room temperature for 2 hours, the reaction mixture was concentrated and extracted with chloroform. The extract was washed with diluted hydrochloric acid and an aqueous sodium bicarbonate solution in order, dried and concentrated to give 150 ml of 4-oxa-2-acetylaminomethyl-isotwistane. $IR\nu_{max}^{Nujol}$ ($cm^{-1}$): 3300, 1660, 1210, 1080, 1040.

Preparative Example 21

4-Oxa-2-carboxymethyl-isotwistane (0.41 g) obtained in Preparative Example 11 was added to 4 ml of thionyl chloride, and the resultant mixture was refluxed. After 2 hours, the reaction mixture was concentrated under reduced pressure to give 0.44 g of the corresponding acid chloride. $IR\nu_{max}^{film}$ ($cm^{-1}$): 1805, 1740.

The thus obtained acid chloride (0.44 g) dissolved in 5 ml of dry benzene was added to diallylamine (406 g) in 10 ml of dry benzene at 5°C. The resultant mixture was allowed to stand at the same temperature for 1 hour and stirred at room temperature for 17 hours. The reaction mixture was concentrated under reduced pressure to give 1.41 g of an oily substance, which was subjected to purification by silica gel column chromatography using chloroform/methanol (97/3) as an eluent to give 0.60 g of 4-oxa-2-N,N-diallylcarbamoylmethyl-isotwistane. $IR\nu_{max}^{film}$ ($cm^{-1}$): 1640, 1415, 1060.

Example 16

Under nitrogen atmosphere, a solution of 4-oxa-2-N,N-diallylcarbamoylmethyl-isotwistane (0.6 g) obtained in Preparative Example 21 in 10 ml of tetrahydrofuran was dropwise added to a dispersion of lithium aluminum hydride (0.1 g) in tetrahydrofuran at room temperature. After completion of the addition, the resultant mixture was refluxed for 3 hours. The reaction mixture was ice-cooled, and water (1 ml), 15 % aqueous sodium hydroxide solution (1 ml) and water (3 ml) were added thereto in order. The mixture was subjected to decantation to remove the organic layer. The residue was washed well with ethyl acetate. The organic phases were combined, washed with a saturated sodium chloride solution and dried. Concentration of the organic phase gave 0.55 g of 4-oxa-2-N,N-diallylaminoethyl-isotwistane as an oily substance. IR$\nu_{max}^{film}$ ($cm^{-1}$): 1640, 1450, 1060.

Example 17

4-Oxa-2-carboxymethyl-isotwistane (0.44 g) obtained in Preparative Example 11 was dissolved in 10 ml of acetone, followed by addition of triethylamine (0.3 g). To the resultant mixture cooled to -15°C, a solution of ethyl chlorocarbonate (0.29 g) in 1 ml of acetone was added, and the reaction mixture was stirred at -20°C for 1 hour. The mixture was further cooled to -30°C, and a solution of sodium azide (0.285 g) in water (5 ml) was added thereto, followed by stirring at the same temperature as above for 1 hour. The reaction mixture was extracted with benzene, and the benzene extract was dried and concentrated under reduced

pressure until the total weight thereof became about 10 ml. The concentrate was added with benzyl alcohol (30 ml) and heated to 100°C. After allowed to stand for 6 hours, the reaction mixture was concentrated under reduced pressure to give a yellow oily substance, which was then subjected to purification by silica gel column chromatography using benzene/ethyl acetate (20/1) as an eluent to give 0.15 g of 4-oxa-2-benzyloxycarbonylaminomethyl-isotwistane. $IR\nu_{max}^{film}$ ($cm^{-1}$): 1700, 1595.

 

CLAIMS:

1. A compound of the formula:

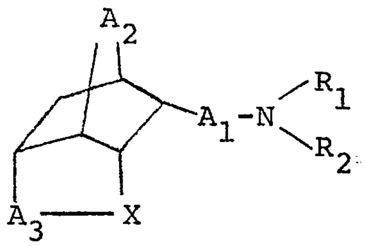

wherein $A_1$ is a single bond, a $C_2$-$C_5$ alkylene group or a $C_1$-$C_5$ alkylidene group, $A_2$ is a $C_2$-$C_3$ alkylene group or a $C_1$-$C_3$ alkylidene group, $A_3$ is a $C_2$-$C_4$ alkylene group or a $C_1$-$C_4$ alkylidene group, X is -O- or $-SO_n-$ (wherein n is 0, 1 or 2), $R_1$ is a hydrogen atom, a $C_1$-$C_5$ alkyl group, a $C_3$-$C_5$ alkenyl group and $R_2$ is a hydrogen atom, a $C_1$-$C_5$ alkyl group, a $C_3$-$C_5$ alkenyl group, a $C_2$-$C_5$ alkanoyl group, a $C_2$-$C_5$ alkoxycarbonyl group, a benzyloxycarbonyl group, or when taken together with the adjacent nitrogen atom, $R_1$ and $R_2$ form a five or six-membered heterocyclic group, or a non-toxic salt thereof.

2. A compound as claimed in claim 1, of the formula:

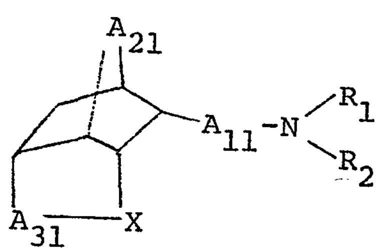

wherein $A_{11}$ is a single bond or a methylene group, $A_{21}$ is a methylene group or an ethylene group, $A_{31}$ is a methylene group or an ethylene group, X, $R_1$ and $R_2$ are each as defined in claim 1, or a non-toxic salt thereof.

3. A compound as claimed in claim 2, of the formula:

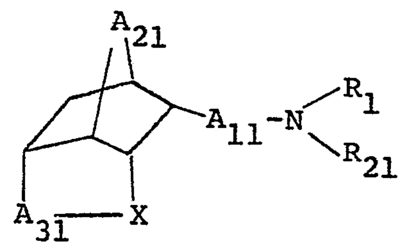

wherein $A_{11}$, $A_{21}$, $A_{31}$ and X are each as stated in claim 2, $R_1$ is a hydrogen atom, a $C_1$-$C_5$ alkyl group, a $C_3$-$C_5$ alkenyl group, and $R_{21}$ is a hydrogen atom, a $C_1$-$C_5$ alkyl group, a $C_3$-$C_5$ alkenyl group or a $C_2$-$C_5$ alkoxycarbonyl group, or a non-toxic salt thereof.

4. A compound as claimed in claim 3, of the formula:

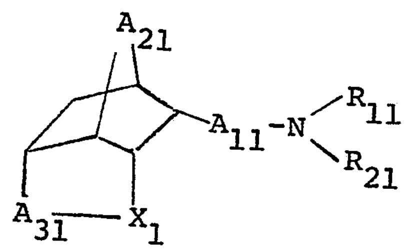

Wherein $A_{11}$, $A_{21}$, $A_{31}$ and $R_{21}$ are each as defined in claim 3, $R_{11}$ is a hydrogen atom or a $C_1$-$C_5$ alkyl group, and $X_1$ is -O- or -S-, or a non-toxic salt thereof.

5. A compound as claimed in claim 4, of the formula:

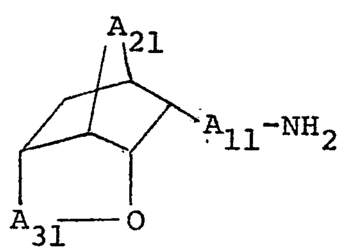

wherein $A_{11}$, $A_{21}$ and $A_{31}$ are each as defined in claim 4, or a non-toxic salt thereof.

6. A process for producing a compound of the formula:

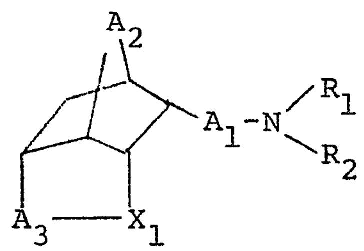

wherein $A_1$, $A_2$, $A_3$, $R_1$ and $R_2$ are each as defined in claim 1 and $X_1$ is -O- or -S-, which comprises reducing

a) a compound of the formula:

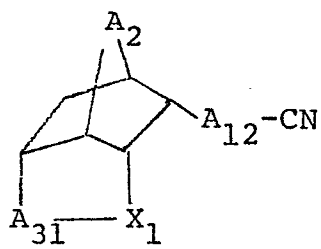

wherein $A_2$, $A_3$ and $X_1$ are each as defined above and $A_{12}$ is a single bond, a $C_2$-$C_4$ alkylene group or a $C_1$-$C_4$ alkylidene group, with a metal hydride compound or hydrogen gas in the presence of a catalyst; or

b) a compound of the formula:

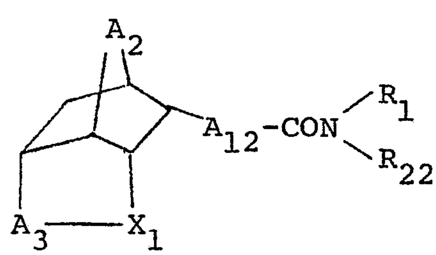

wherein $A_{12}$, $A_2$, $A_3$, $R_1$ and $X_1$ are each as defined above and $R_{22}$ is a hydrogen atom, a $C_1$-$C_5$ alkyl group, a $C_3$-$C_5$ alkenyl group, or when taken together with the adjacent nitrogen atom, $R_1$ and $R_{22}$ may form a five or six-membered heterocyclic group, with a metal hydride compound; or

c) a compound of the formula:

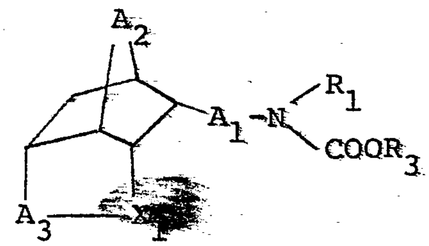

wherein $A_1$, $A_2$, $A_3$, $R_1$ and $X_1$ are each as defined above and $R_3$ is a $C_1$-$C_4$ alkyl group, with a metal hydride compound; or

d) a compound of the formula:

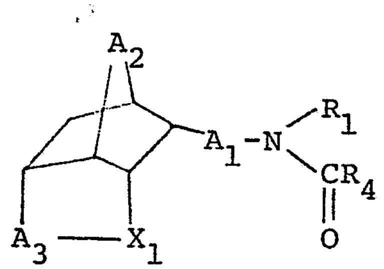

wherein $A_1$, $A_2$, $A_3$, $R_1$ and $X_1$ are each as defined above and $R_4$ is a hydrogen atom, a $C_1$-$C_4$ alkyl group or a $C_2$-$C_4$ alkenyl group, with a metal hydride compound; or

e) a compound of the formula:

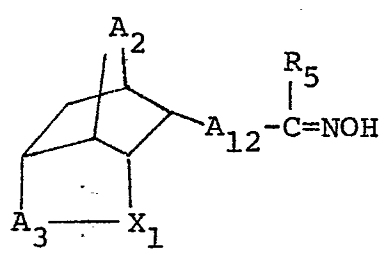

wherein $A_2$, $A_3$, $A_{12}$ and $X_1$ are each as defined above and $R_5$ is a hydrogen atom or a $C_1$-$C_4$ alkyl group, with a metal hydride compound or hydrogen gas in the presence of a catalyst; or

f) a compound of the formula:

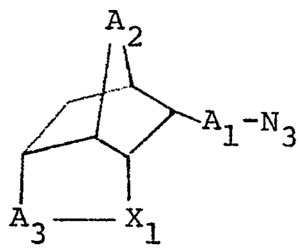

wherein $A_1$, $A_2$, $A_3$ and $X_1$ are each as defined above, with a metal hydride compound or hydrogen gas in the presence of a catalyst; or

g) a compound of the formula:

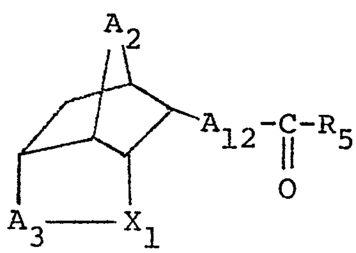

wherein $A_2$, $A_3$, $A_{12}$, $R_5$ and $X_1$ are each as defined above, with a reducing agent in the presence of an amine compound of the formula:

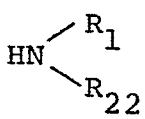

wherein $R_1$ and $R_{22}$ are each as defined above.

7. A process for producing a compound of the formula:

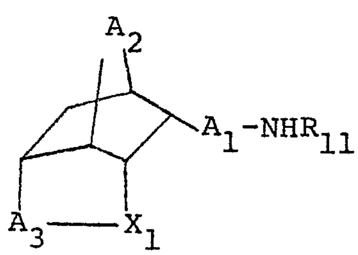

wherein $A_1$, $A_2$ and $A_3$ are each as defined in claim 1, $X_1$ is -O- or -S- and $R_{11}$ is a hydrogen atom or a $C_1$-$C_5$ alkyl group, which comprises debenzylation and decarbonylation of a compound of the formula:

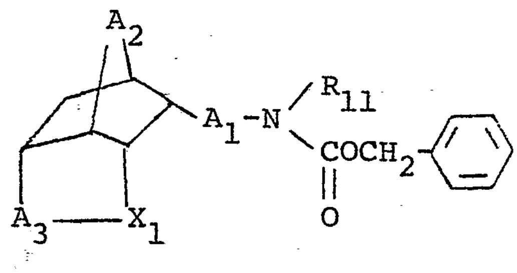

wherein $A_1$, $A_2$, $A_3$, $R_{11}$ and $X_1$ are each as defined above.

8. A process for producing a compound of the formula:

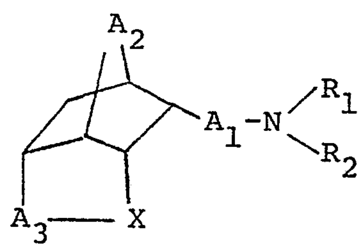

wherein $A_1$, $A_2$, $A_3$, X, $R_1$ and $R_2$ are each as defined in claim 1, which comprises reacting a compound of the formula:

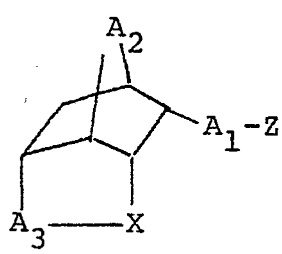

wherein $A_1$, $A_2$, $A_3$ and X are each as defined above and Z is the residue of a strong acid, with an amine compound of the formula:

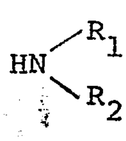

wherein $R_1$ and $R_2$ are each as defined above.

9. A process for producing a compound of the formula:

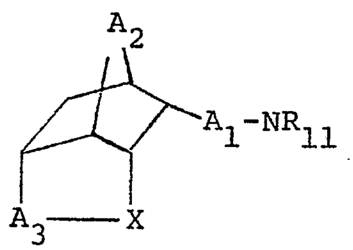

wherein $A_1$, $A_2$, $A_3$ and X are each as defined in claim 1 and $R_{11}$ is a hydrogen atom, or a $C_1$-$C_5$ alkyl group, which comprises hydrolyzing a compound of the formula:

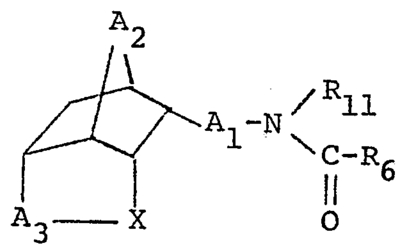

wherein $A_1$, $A_2$, $A_3$, $R_{11}$ and X are each as defined above and $R_6$ is a hydrogen atom, a $C_1$-$C_4$ alkyl group, a $C_1$-$C_3$ haloalkyl group, a $C_1$-$C_4$ alkoxy group or a benzyloxy group.

10. A process for producing a compound of the formula:

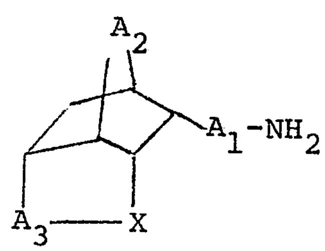

wherein $A_1$, $A_2$, $A_3$ and X are each as defined in claim 1, which comprises hydrolyzing a compound of the formula:

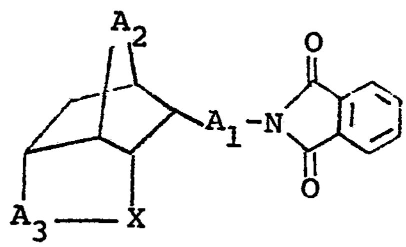

wherein $A_1$, $A_2$, $A_3$ and X are each as defined above.

11. A process for producing a compound of the formula:

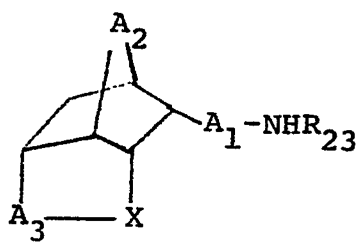

wherein $A_1$, $A_2$, $A_3$ and X are each as defined in claim 1 and $R_{23}$ is a hydrogen atom, a $C_2$-$C_5$ alkoxycarbonyl group or a benzyloxycarbonyl group, which comprises treatment of

a) a compound of the formula:

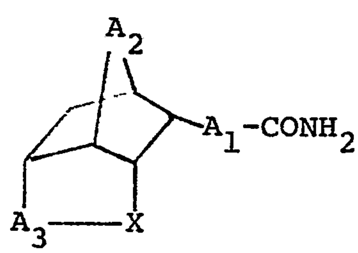

wherein $A_1$, $A_2$, $A_3$ and X are each as defined above, with alkali hypohalite, followed by hydrolysis or treatment of the resulting rearrangement product (an isocyanate) with an alcohol compound of the formula: $R_7$-OH (wherein $R_7$ is a $C_1$-$C_4$ alkyl group or a benzyl group); or

b) a compound of the formula:

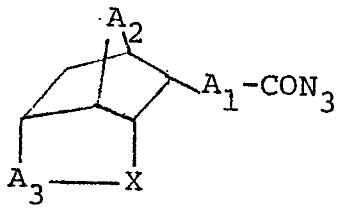

wherein $A_1$, $A_2$, $A_3$ and X are each as defined above, under heating, followed by hydrolysis or treatment of the resulting rearrangement product (an isocyanate) with an alcohol compound of the formula: $R_7$-OH (wherein $R_7$ is as defined above);

c) a compound of the formula:

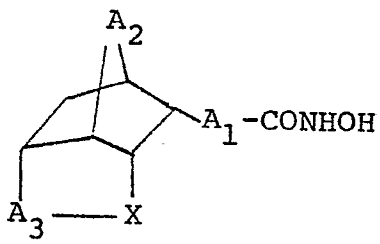

wherein $A_1$, $A_2$, $A_3$ and X are each as defined above, under heating with or without a base, followed by hydrolysis or treatment of the resulting rearrangement product (an isocyanate) with an alcohol compound of the formula: $R_7$-OH (wherein $R_7$ is as defined above).

12. A process for producing a compound of the formula:

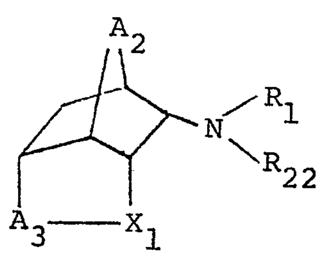

wherein $A_2$, $A_3$, $R_1$ and $R_{22}$ are each as defined in claim 1 and $X_1$ is -O- or -S-, which comprises reducing

a) a compound of the formula:

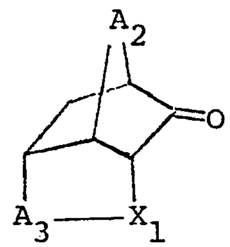

wherein $A_2$, $A_3$ and $X_1$ are each as defined above, with a reducing agent in the presence of an amine of the formula:

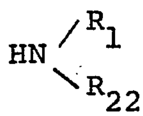

wherein $R_1$ and $R_{22}$ are each as defined above; or

b) a compound of the formula:

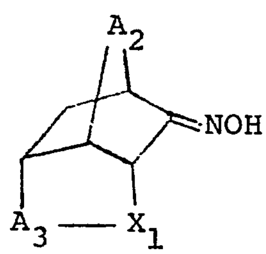

wherein $A_2$, $A_3$ and $X_1$ are each as defined above, with a metal hydride compound or hydrogen gas in the presence of a catalyst.

13. A process for producing a compound of the formula:

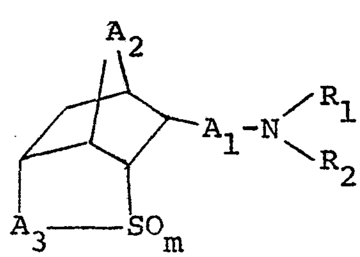

wherein $A_1$, $A_2$, $A_3$, $R_1$ and $R_2$ are each as defined in claim 1 and m is 1 or 2, which comprises oxidizing a

0040944

compound of the formula:

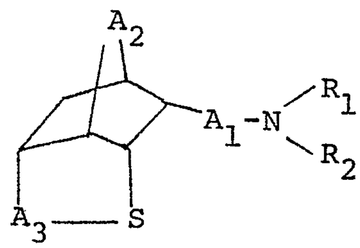

wherein $A_1$, $A_2$, $A_3$ and $R_2$ are each as defined above.

14. A pharmaceutical composition which comprises as an active ingredient a pharmaceutically effective amount of at least one compound as claimed in claim 1, together with at least one pharmaceutical inert carrier or diluent.

15. Use of the compound claimed in claim 1 as a medicine.

0040944

European Patent Office

# EUROPEAN SEARCH REPORT

Application number
EP 81 30 2197

## DOCUMENTS CONSIDERED TO BE RELEVANT

| Category | Citation of document with indication, where appropriate, of relevant passages | Relevant to claim |
|---|---|---|
| | EP - A - 0 006 283 (SUMITOMO)<br>* claims *<br>----- | 1,6-15 |

CLASSIFICATION OF THE APPLICATION (Int. Cl.3)

C 07 D 307/93
333/78
A 61 K 31/34
31/38

TECHNICAL FIELDS SEARCHED (Int. Cl.3)

C 07 D 307/93
333/78
A 61 K 31/34
31/38
C 07 D 307/00
333/50

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 26-08-1981 | CHOULY |

EPO Form 1503.1 06.78